# EUROPEAN PATENT SPECIFICATION

(11) **EP 1 007 511 B1**
(45) Date of publication and mention of the grant of the patent: **02.07.2003**
(21) Application number: 98943349.5
(22) Date of filing: 24.08.1998
(51) Int. Cl.: C07D 205/08, C07F 7/18

(54) **PREPARATION OF BETA-METHYL CARBAPENEM INTERMEDIATES**
HERSTELLUNG VON BETA-MEHTYLCARBAPENEM-ZWISCHENPRODUKTEN
PREPARATION D'INTERMEDIAIRES DE BETA-METHYL CARBAPENEM

(30) Priority: 27.08.1997 US 58002 P; 16.01.1998 GB 9801003
(43) Date of publication of application: 14.06.2000
(73) Proprietor: MERCK & CO., INC., Rahway, New Jersey 07065 (US)
(72) Inventor: CHOI, Woo-Baeg, Rahway, NJ 07065 (US); LEE, Jaemoon, Rahway, NJ 07065 (US); LYNCH, Joseph, E., Rahway, NJ 07065 (US); REIDER, Paul, J., Rahway, NJ 07065 (US); VOLANTE, Ralph, P., Rahway, NJ 07065 (US)
(74) Representative: Hiscock, Ian James
(86) International application number: US9817510
(87) International publication number: WO99010323

(56) References cited:
- GB-A- 2 183 236
- US-A- 4 960 879
- T. MURAYAMA ET AL.: "A simple and highly diastereoselective synthesis of a 1-beta-methylcarbapenem key intermediate by deallyloxycarbonylation using palladium complexes" TETRAHEDRON LETTERS, vol. 35, no. 15, 1994, pages 2271-2274, XP002167358 OXFORD GB
- W.-B. CHOI ET AL: "A stereoselective synthesis of a key 1-beta-methylcarbapenem intermediate via a diastereoselective decarboxylation" TETRAHEDRON LETTERS, vol. 35, no. 15, 1994, pages 2275-2278, XP002167359 OXFORD GB
- UYEO et al., "Practical, Stereocontrolled Synthesis of 2-Functionalized-Methyl-1beta- Methylcarbapenems", TETRAHEDRON LETTERS, 1994, Vol. 35, No. 25, pages 4377-4378, XP002914924
- SHIOZAKI et al., "Synthesis and Biological Activities of New Carbapenem Derivatives", J. ANTIBIOTICS, January 1984, Vol. 37, No. 1, pages 57-62, XP002914925
- HU et al., "Synthesis of 2-(Hydroxymethyl)-1beta-Methylcarbapenem. Chemoselective Organometallic Addition to 3-Substituted 4-[(R)-1-Carboxyethyl]Azetidin-2-One", J. ORGANIC CHEMISTRY, May 1998, Vol. 63, No. 5, pages 1719-1723, XP002914926

## Description

### BACKGROUND OF THE INVENTION

The invention disclosed herein concerns a beta-methyl-hydroxymethyl ketone, and process for synthesis thereof, which is a key intermediate used in making beta-methyl carbapenems. 1-beta methyl carbapenem antibiotics, are particularly well known for treating a broad spectrum of gram-negative and gram-positive bacterial infections. See for example U.S. 4,962,103 issued October 9, 1990; U.S. 4,933,333; U.S. 4,943,569; U.S. 5,122,604; U.S. 5,034,384 and U.S. 5,011,832.

Numerous routes to beta-methyl carbapenem intermediates of formula 6 have been cited in the literature:

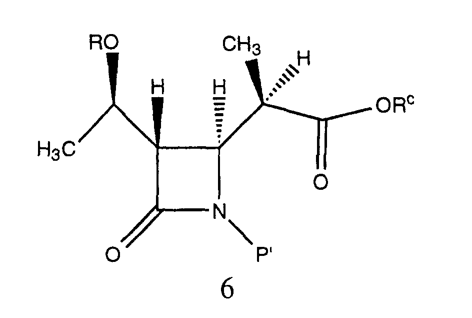

*Tetrahedron Letters,* Vol. 26, No. 39, pp 4739-4742, 1985; *J. Am. Chem. Soc*. 1986, 108, 4673-3675; *Tetrahedron Letters,* Vol. 27, No. 19, pp 2149-2152, 1986; *Can. J. Chem.* 65, 2140 (1987); *Can. J. Chem.* 66, 1400 (1988); *Chemistry Letters,* pp 445-448, 1989; *Tetrahedron Letters,* Vol. 31, No. 2, pp 271-274, 1990; *Tetrahedron Letters,* Vol 31, No. 4, pp 549-552, 1990; *J. Org. Chem.* 1992, 57, 2411-2418; and the like.

Previous methods of stereoselective preparation of beta-methyl carbapenems include:
(1) hydrogenation of a 4-(2-propenyl) substituted azetidinone.
(2) stereoselective protonation of an enolate ion
(3) reaction of 4-acetoxyazetidinone with a chiral enolate.

These methods require difficult multi-step preparation, tedious manipulation of highly reactive intermediates at low temperature, or use of expensive reagents.

The instant invention discloses an efficient process for the synthesis of beta-methyl intermediates with high stereoselectivity from readily available starting materials.

### SUMMARY OF THE INVENTION

The instant invention relates to a process for the preparation of a compound of formula 5: 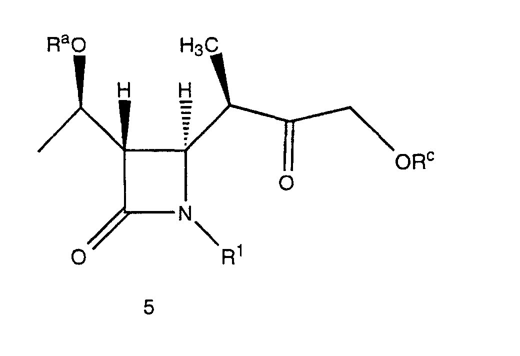 wherein:
- R^{a} is:: (1) hydrogen,
(2) C₁₋₄ alkyl or
(3) a hydroxy protecting group; and
- R^{b} and R^{c} are independently:: tri-organo-silyl, including tri-C₁₋₆ alkyl silyl, phenyl di-C₁₋₆ alkyl silyl, and diphenyl mono C₁₋₆ alkyl silyl including *tert*-butyl-dimethylsilyl, hexyldimethylsilyl and isopropyl dimethylsilyl; straight and branched lower alkyl having from 1 to 10 carbon atoms; alkenyl or alkynyl, having from 2 to 10 carbon atoms; cycloalkyl having from 3 to 6 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; aryl, such as phenyl and naphthyl; aralkyl such as benzyl, phenethyl and the like; heterocyclyl (saturated and unsaturated) comprising mono- and bicyclic structures having from 5 to 10 ring atoms, wherein one or more of the heteroatoms is selected from oxygen, nitrogen, or sulfur, such as thiophene, imidazolyl, tetrazolyl,furyl and the like; heterocycloalkyl which comprises the immediately preceding heterocyclyl moieties and the alkyl moiety comprises from 1-10 carbon atoms; substituted species of the above named radicals wherein the substituents are selected from the group consisting of amino, hydroxyl, cyano, carboxyl, nitro, chloro, bromo, or fluoro; lower alkoxy having from 1 to 6 carbon atoms; mercapto; perhaloloweralkyl such as trifluoromethyl; lower alkylthio; guanidino; amidino; sulfamoyl; N-substituted sulfamoyl, amidino, and guanidino wherein the N-substituent is lower alkyl having from 1 to 6 carbon atoms or aryl having 6-10 carbon atoms; COR or CO₂R, wherein R is lower alkyl having from 1 to 6 carbon atoms or aryl having 6-10 carbon atoms; wherein lower alkyl is C₁₋₆ alkyl and wherein R^{b} and R^{c} may be the same or different, but R^{b} must be removable in the presence of R^{c}; and
R¹ is an alkylsilyl protecting group corresponding to the silylating agent employed;
comprising: combining the ketoester of formula 4: 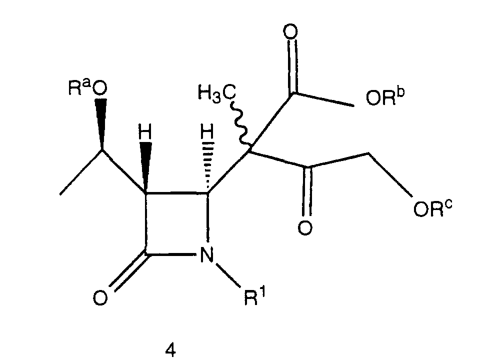 wherein R^{a}, R^{b}, R^{c} and R¹ are described above;
with an acid and a first catalyst at a temperature of 0°C to 50°C with 0-3447.38 kPa 0-500 psi H₂, to give a compound of formula 5.

This invention also relates to a compound of formula I: 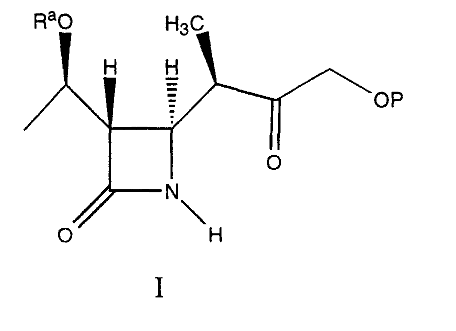 wherein R^{a} and P are described below and a method of making a compound of formula I.

Other aspects of the invention will be realized upon review of the application as a whole.

### DETAILED DESCRIPTION OF THE INVENTION

The instant invention relates to an efficient process for the preparation of beta-methyl intermediates with high stereoselectivity from readily available starting materials. These intermediates can be used to make carbapenem antibiotics as illustrated in Imuta, et al., Chem. Pharm. Bull., 39(3) 672-678 (1991) and Imuta, et al., Chem. Pharm. Bull., 39(3) 663-671 (1991).

In one embodiment of this invention, a process for synthesizing a compound of formula 6: 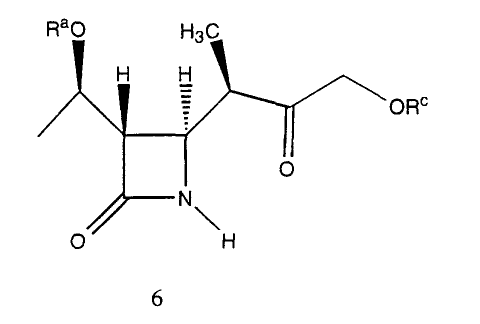 wherein:
- R^{a} is:: (1) hydrogen,
(2) C₁₋₄ alkyl or
(3) a hydroxy protecting group; and
- R^{b} and R^{c} are independently:: tri-organo-silyl, including tri-C₁₋₆ alkyl silyl, phenyl di-C₁₋₆ alkyl silyl, and diphenyl mono C₁₋₆ alkyl silyl including *tert*-butyl-dimethylsilyl, hexyldimethylsilyl and isopropyl dimethylsilyl; straight and branched lower alkyl having from 1 to 10 carbon atoms; alkenyl or alkynyl, having from 2 to 10 carbon atoms; cycloalkyl having from 3 to 6 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; aryl, such as phenyl and naphthyl; aralkyl such as benzyl, phenethyl and the like; heterocyclyl (saturated and unsaturated) comprising mono- and bicyclic structures having from 5 to 10 ring atoms, wherein one or more of the heteroatoms is selected from oxygen, nitrogen, or sulfur, such as thiophene, imidazolyl, tetrazolyl, furyl and the like; heterocycloalkyl which comprises the immediately preceding heterocyclyl moieties and the alkyl moiety comprises from 1-10 carbon atoms; substituted species of the above named radicals wherein the substituents are selected from the group consisting of amino, hydroxyl, cyano, carboxyl, nitro, chloro, bromo, or fluoro; lower alkoxy having from 1 to 6 carbon atoms; mercapto; perhaloloweralkyl such as trifluoromethyl; lower alkylthio; guanidino; amidino; sulfamoyl; N-substituted sulfamoyl, amidino, and guanidino wherein the N-substituent is lower alkyl having from 1 to 6 carbon atoms or aryl having 6-10 carbon atoms; COR or CO₂R, wherein R is lower alkyl having from 1 to 6 carbon atoms or aryl having 6-10 carbon atoms; wherein lower alkyl is C₁₋₆ alkyl and wherein R^{b} and R^{c} may be the same or different, but R^{b} must be removable in the presence of R^{c}; and
comprising: combining the ketoester of formula 4: 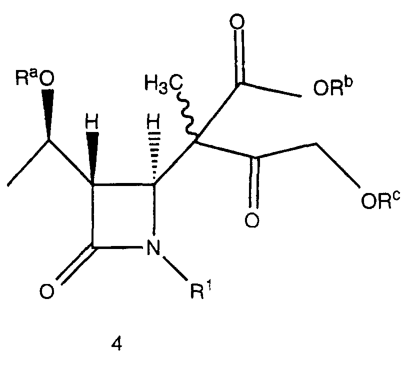 wherein R^{a}, R^{b}, and R^{c} are defined above and R¹ is an alkylsilyl protecting group corresponding to the silylating agent employed;
with an acid and a first catalyst at a temperature of 0°C to 50°C with 0-344 7.38 kPa 0-500 psi H₂ to give a compound of formula 5: 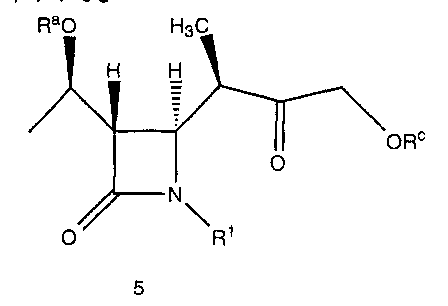 and selectively desilylating with a desilylating agent at a temperature of -10°C to 50°C to yield the compound of formula 6 is described.

In still another embodiment of the invention, a process for synthesizing a compound of formula I is described: 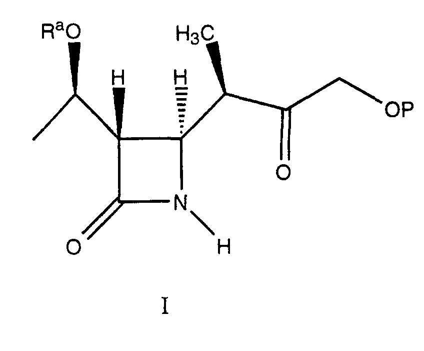 wherein:
- R^{a} and P are independently:: (1) hydrogen,
(2) C₁₋₄ alkyl or
(3) a hydroxy protecting group; and
comprising:
(a) reacting a ketoester of formula 1: 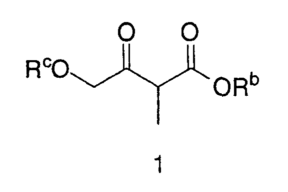 wherein
   - R^{b} and R^{c} are independently:: H, tri-organo-silyl, including tri-C₁₋₆ alkyl silyl, phenyl di-C₁₋₆ alkyl silyl, and diphenyl mono C₁₋₆ alkyl silyl including *tert*-butyl-dimethylsilyl, hexyldimethylsilyl and isopropyl dimethylsilyl; straight and branched lower alkyl having from 1 to 10 carbon atoms; alkenyl or alkynyl, having from 2 to 10 carbon atoms; cycloalkyl having from 3 to 6 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; aryl, such as phenyl and naphthyl; aralkyl such as benzyl, phenethyl and the like; heterocyclyl (saturated and unsaturated) comprising mono- and bicyclic structures having from 5 to 10 ring atoms, wherein one or more of the heteroatoms is selected from oxygen, nitrogen, or sulfur, such as thiophene, imidazolyl, tetrazolyl,furyl and the like; heterocycloalkyl which comprises the immediately preceding heterocyclyl moieties and the alkyl moiety comprises from 1-10 carbon atoms; substituted species of the above named radicals wherein the substituents are selected from the group consisting of amino, hydroxyl, cyano, carboxyl, nitro, chloro, bromo, or fluoro; lower alkoxy having from 1 to 6 carbon atoms; mercapto; perhaloloweralkyl such as trifluoromethyl; lower alkylthio; guanidino; amidino; sulfamoyl; N-substituted sulfamoyl, amidino, and guanidino wherein the N-substituent is lower alkyl having from 1 to 6 carbon atoms or aryl having 6-10 carbon atoms; COR or CO₂R, wherein R is lower alkyl having from 1 to 6 carbon atoms or aryl having 6-10 carbon atoms; wherein lower alkyl is C₁₋₆ alkyl and wherein R^{b} and R^{c} may be the same or different, but R^{b} must be removable in the presence of R^{c};
   with a compound of formula 2: 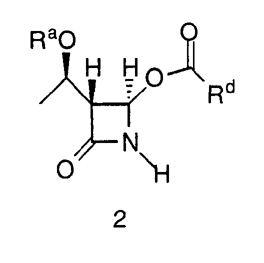 wherein R^{a} is defined above, and R^{d} is C₁₋₄ alkyl;
   in the presence of a first base at a temperature of 25° C to 60° C, preferably 35°C to 50°C, to produce a ketoester adduct of formula 3: 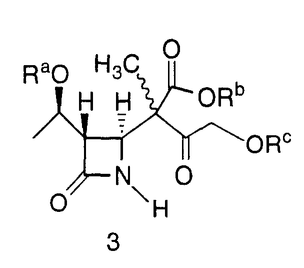
(b) combining the compound of formula 3 with a silylating agent and a second base to produce the ketoester of formula 4: 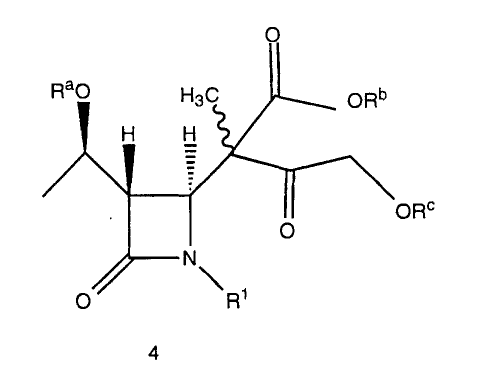 wherein R^{a}, R^{b}, and R^{c} are defined above and R¹ is an alkylsilyl protecting group corresponding to the silylating agent employed;
(c) combining the isolated ketoester of formula 4 with an acid and a first catalyst at a temperature of 0°C to 50°C with 0-3447.38 kPa 0-500 psi H₂, to give a compound of formula 5: 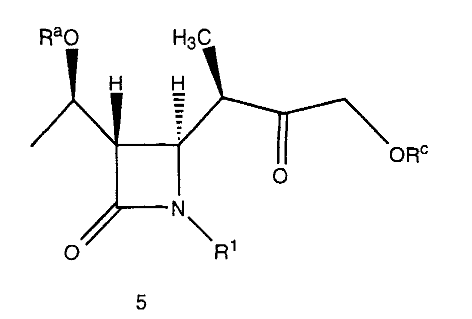
(d) selectively desilylating with a desilylating agent at a temperature of -10°C to 50°C to yield the compound of formula 6: 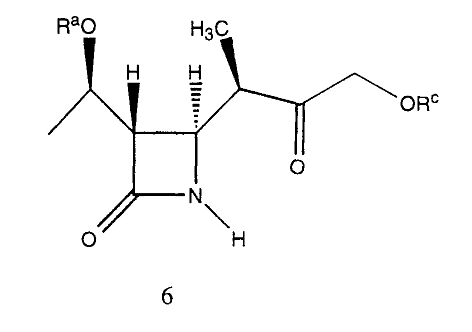
(e) dissolving the compound of formula 6 in an alcohol and hydrogenating at 206.84-379.21 kPa 30 to 55 psi H₂ in the presence of a second catalyst at a temperature of 0°C to 100°C, to yield compound I and
(f) purifying and isolating compound I.

A preferred aspect of this embodiment is realized when R^{a} is a hydroxy protecting group, preferably t-butyldimethylsilyl, and R^{b} and R^{c} are independently selected from the group consisting of benzyl, substituted benzyl, ethoxycarbonyl, t-butyloxycarbonyl, benzyloxycarbonyl, allyloxycarbonyl, fluorenylmethyloxycarbonyl, t-butyldimethylsilyl, and isopropyldimethylsilyl, more preferably of benzyl, substituted benzyl, ethoxycarbonyl, or t-butyloxy carbonyl, wherein the benzyl is substituted with 1 to 3 groups of C₁₋₆ alkyl, NO₂, halogen and the like and all other variables are as described above.

Some of the intermediate compounds synthesized in the present invention occur as diastereomers. The processes of synthesizing all such isomers are included in the present invention.

The desired ketoester of formula 1 can be prepared from corresponding esters, using the standard Claisen condensation technique, as described, for example, in Organic Chemistry, L.G. Wade, Jr., Prentice Hall 1991, pp. 1012-1019.

Hydrogenation is carried out with 0-3447.38 kPa 0-500 psi H₂, preferably 68.95-689.48 kPa 10-100 psi, and more preferably 137.90-275.79 kPa 20-40 psi H₂.

When a functional group is termed "protected", this means that the group is in modified form to preclude undesired side reactions at the protected site. Suitable protecting groups for the compounds of the present invention will be recognized from the present application taking into account the level of skill in the art, and with reference to standard textbooks, such as Greene, T. W. et al. Protective Groups in Organic Synthesis Wiley, New York (1991). Examples of suitable protecting groups, are: tri-organo-silyl, including tri-C₁₋₆ alkyl silyl, phenyl di C₁₋₆ alkyl silyl, and diphenyl mono C₁₋₆ alkyl silyloxy including *tert*-butyldimethylsilyl, substituted and unsubstituted benzyl, allyl, triethylsilyl, carbonate esters including t-butyloxycarbonyl, , o-nitrobenzyloxycarbonyl, p-nitrobenzyloxycarbonyl, 2,2,2-trichloroethyloxycarbonyl, benzoylxycarbonyl, allyloxycarbonyl, and fluorenylmethyloxycarbonyl, preferably t-butyldimethyl silyl.

As used herein, "alkyl" is intended to include branched, cyclic and straight-chain saturated aliphatic hydrocarbon groups having the specified number of carbon atoms. Examples of cycloalkyl groups include cyclopropyl, cyclobutyl, cyclopentyl, cyclohexyl and the like.

As used herein, "aryl" is intended to include aryls and heteroaryls, both substituted and unsubstituted, which are defined as carbazolyl, furyl, thienyl, pyrrolyl, isothiazolyl, imidazolyl, isoxazolyl, thiazolyl, oxazolyl, pyrazolyl, pyrazinyl, pyridyl, pyrimidyl, purinyl or quinolinyl as well as aromatic rings e.g., phenyl, substituted phenyl and like groups as well as rings which are fused, e.g., naphthyl and the like. Substitution can be 1 to 3 groups of C₁₋₆ alkyl, hydroxy, halogen, carbonyl, CO₂, NO₂, OC₁₋₆ alkyl; SC₁₋₆ alkyl, N(C₁₋₆ alkyl)₂ and the like.

For purposes of this specification, suitable solvents are defined to include a broad spectrum of non-reacting solubilizing agents including: aromatic solvents such as benzene, toluene and xylene; etheral solvents such as diethyl ether, di-n-butyl and diisopentyl ethers, anisole, cyclic ethers such as tetrahydropyran, 4-methyl-1,3-dioxane, dihydropyran, tetrahydrofurfuryl, methyl ether, ethyl ether, furan, 2-ethoxytetrahydrofuran and tetrahydrofuran (THF); ester solvents including ethyl and isopropyl acetate; halo carbon solvents including mono or dihalo C₁₋₄ alkyl such as dichloromethane; alcohols, including C₁₋₆ alkanol; C₆₋₁₀ linear, branched or cyclic hydrocarbon solvents including hexane; and nitrogen containing solvents including N,N-dimethylacetamide, N,N-dimethylformamide (DMF), N-ethylpyrrolidinone, N-methylpyrrolidinone, and acetonitrile. Preferable solvents are alcohol, EtOAc, isopropyl acetate, hexane, toluene, dichloromethane, THF, DMF, and CH₃CN.

Suitable first and second bases are intended to include carbonates, including alkali carbonates such as the potassium and calcium carbonates, diazabicycloundecane (DBU) and tri C₁₋₆ alkyl amines, including diisopropylethylamine, triethylamine, dimethylethylamine, dimethylpentylamine and the like. Preferably, the first base is K₂CO₃ or DBU. A preferred second base is Et₃N or DBU.

Suitable silylating agents are intended to include trialkylsilylchlorides, triakylsilyliodides, and triflates. In a preferred aspect of this invention, the silylating agent employed is chosen from the group comprising trimethylsilyltriflate (TMSOTf), t-butyldimethylsilyltriflate (TBSOTf), triethylsilyltriflate (TESOTf), or TBSCl/NaI. In a more preferred aspect of this invention, the silylating agent is TBSOTf or TBSCl/NaI.

Suitable acids are intended to include mono-, di-, and tri-carboxylic acids, preferably acetic or formic acid.

Suitable desilylating agents are intended to include NaOH, KOH, N-desilylating agents such as tetrabutylammonium fluoride (TBAF), 2-mercaptopyridine N-oxide. Preferably, the desilylation is carried out using an N-desilylating agent such as TBAF in the presence of dichloromethane. Alternatively, N-desilylation is carried out with 2-mercaptopyridine N-oxide as desilylating agent in DMF.

Suitable alcohols are intended to include C1-6 alcohols such as methanol, ethanol, 1-propanol, butanol, pentanol, 2-propanol and the like. In a preferred aspect of this invention, the alcohol employed is methanol or ethanol.

Suitable first and second catalysts are intended to include Pd/C, Pd(OH)₂/C, or (Ra)Ni (Raney nickel). In a preferred aspect of this invention, Pd/C or Pd(OH)₂/C is employed as the first and second catalyst.

In yet another aspect of this invention, a compound of formula I is described: 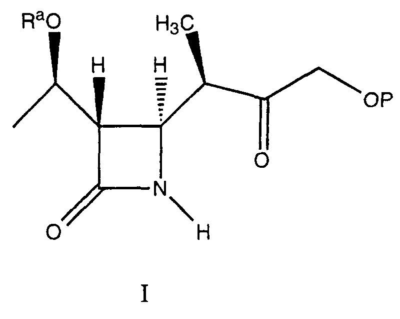 wherein R^{a} and P are:
(1) hydrogen,
(2) C₁₋₄ alkyl, or
(3) a hydroxy protecting group.
In a preferred embodiment of this aspect, R^{a} is t-butyldimethylsilyl and P is hydrogen, benzyl, ethoxycarbonyl or t-butyloxycarbonyl.

The present invention is illustrated by the following nonlimiting reaction scheme and examples:

### REACTION SCHEME

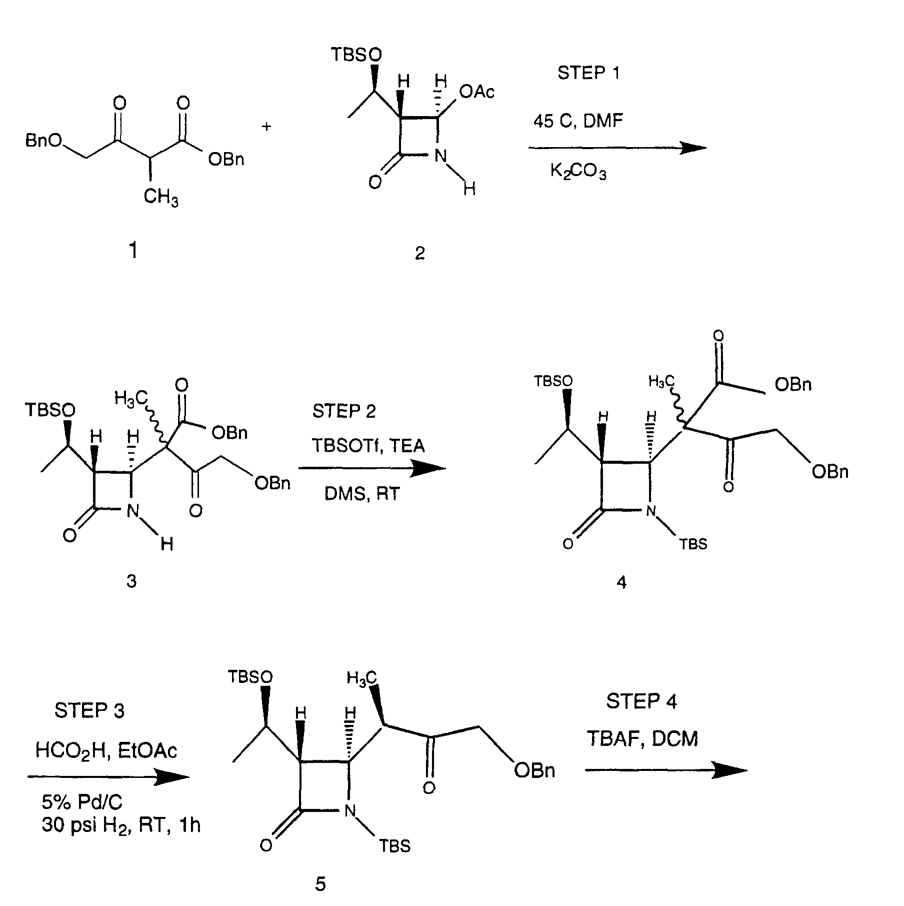

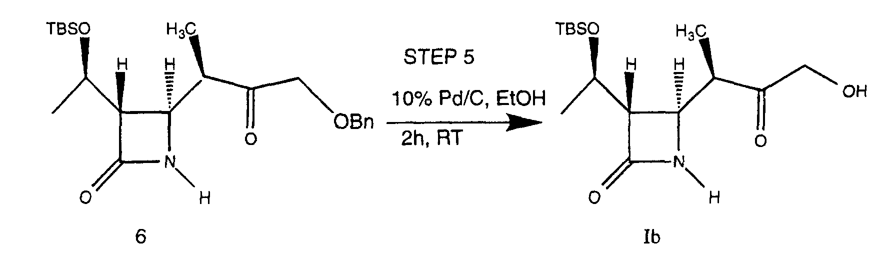

### EXAMPLES

Examples provided are intended to assist in a further understanding of the invention. Particular materials employed, species and conditions are intended to be further illustrative of the invention and not limitative of the reasonable scope thereof.

### Preparation of the Sidechain KetoEster 1

Benzyl alcohol (54 mL, 500 mmol) was mixed with triethyl amine (86 mL, 600 mmol) in dichloromethane (800 mL). The mixture was cooled to 0°C and propionyl chloride (46 mL, 500 mmol) was added dropwise. Internal temperature was maintained below 10°C. The mixture was then aged for 1hr at 0°C and quenched with water (500 mL). The organic layer was separated, washed with water (300 mL), 2N HCl (300 mL), and concentrated to dryness to give 80 g of a slightly yellow oil. This crude benzyl propionate was used in the upcoming step.

NaH (5.6g, 60% oil suspension) was washed with hexanes (50mL) and the wash was decanted. The NaH slurry was then dissolved in a THF/DMF mixture (140mL/35mL) and cooled to 0°C. Methyl glycolate (10 mL, 127 mmol) was added dropwise at 0°C and the mixture was aged for 15 min. Benzyl bromide was then added dropwise at 0°C, and the mixture was allowed to warm up to room temperature ("RT") over 4 hrs. The reaction was quenched with NH₄Cl aq. solution and extracted with 9:1 hexane: ethyl acetate mixture (200 mL). The organic layer was washed with water and concentrated to dryness. This crude methyl *O*-benzyl-glycolate was used in the next step.

Diisopropylamine (14.4 mL, 100 mmol) was dissolved in THF (300 mL) and the mixture was cooled to -40°C. n-BuLi solution (40 mL, 2.5 M in hexane) was added dropwise at -40 to -20°C. The mixture was aged at -70°C for 1 hour. Benzyl propionate from the previous reaction (16g, 100 mmol) was added dropwise to the LDA solution at <-70°C and the mixture was aged at -70°C for 1 hour. Methyl *O*-benzyl-glycolate from the previous reaction (8.8g, 50 mmol) was added to the enolate solution at <-70°C, and the mixture was aged at -70°C for 1 hour. The reaction was quenched with NH₄Cl aq. solution (300 mL) and extracted with ethyl acetate (500 mL). The organic layer was separated, washed with NH₄Cl aq. solution (300 mL), water (300 mL), and concentrated to dryness. The resulting oil contained ca. 1:1 mixture of benzyl propionate and the ketoester of formula 1. This crude product was purified by silica gel chromatography eluting with 10:90 ethyl acetate:hexanes, or was used directly in Step 1 and the coupled product later purified by silica gel chromatography using 20:80 ethyl acetate:hexanes mixture. Yield for the Claisen coupling is estimated to be 80% from NMR.

### Preparation of Ketoester Adduct 3 - Step 1

The crude ketoester (formula 1) from the above reaction (approx. 12g, 40 mmol) and the acetoxyazetidinone (compound 2) (15g, 50 mmol) were dissolved in DMF (100 mL). Potassium carbonate (13.9g, 100 mmol) was added and the mixture aged for 1 hour at 45-50°C. The mixture was then cooled to RT, and quenched with water (300 mL). The mixture was extracted with ethyl acetate (200 mL). The organic layer was separated, washed with 0.2N HCl (200mL x 2) aq. solution and water (100 mL), and concentrated to an oil. Further purification of the oil by silica gel chromatography (20:80 ethyl acetate:hexanes) gave 17.3 g of a 2:1 mixture of diastereomers of the ketoester adduct (formula 3) (32.5 mmol).

### Preparation of bis-TBS Ketoester - Step 2

The lactam (formula 3) (8.2 g, 15.4 mmol) was dissolved in dry DMF (30mL), to which triethylamine (4.5 mL, 30.9 mmol, 2 eq.) and TBSOTf (4.3 mL, 18.5 mmol, 1.2 eq.) were added. The mixture was aged for 1 hour at RT. The reaction mixture was then quenched with water (150 mL) and extracted with ethyl acetate (300 mL). The organic layer was washed with 0.2N HCl aq. solution (100mL x 2) and water (100mL), and concentrated to dryness to give a colorless oil (10.2g). In the case where TBSCl is employed, triethylamine (4.5 mL, 1mmol, 2 eq.), TBSCl (4.52g, 30 mmol), and NaI (4.50g, 30 mol) are added to the lactam (8.2g, 15.4 mol) in dry DMF (30 mL). The mixture is aged at 60 C for 4 hours and the procedure above is followed.

### Preparation of Compound 5 via Decarboxylation - Step 3

The crude ketoester (compound 4)(10.2g, 15 mmol) was dissolved in ethyl acetate (150 mL). Formic acid (2.1 mL, 45 mmol, 3eq.) and the catalyst Pd/C (500 mg, 5w%) were added and the mixture was hydrogenated at 206.84 kPa 30 psi H₂ pressure for 1 hour. The mixture was filtered through a celite pad and the pad was washed with additional ethyl acetate (200 mL) The combined filtrate was washed with saturated sodium bicarbonate solution (50mL) and water (100mL) and concentrated to an oil. Further purification of the resulting oil by silica gel chromatography using 10:90 ethyl acetate:hexanes mixture gave 6.2 g of a colorless oil. (12.1 mmol).

### N-Desilylation to Compound 6 - Step 4

The N-TBS lactam (compound 5)(3.0g, 5.9 mmol) was dissolved in dichloromethane (50mL). The solution was cooled to -5°C and TBAF solution (TBAF in THF 1M, 6 mL, 6 mmol) was added dropwise. The mixture was aged for 1h at -5°C to 0°C. The reaction was quenched with saturated sodium bicarbonate solution. The organic layer was separated, washed with water (30mL x 2), and concentrated to dryness. The resulting oil was purified by silica gel chromatography with a 30:70 ethyl acetate:hexanes mixture to give 1.6g of an oil.

### O-debenzylation of Compound 6 - Step 5

The benzyl ether (formula 6)(0.4g, 1 mmol) was dissolved in ethanol and hydrogenolysed using Pd-C catalyst (40mg, 10w%) at 310,27 kPa 45 psi hydrogen pressure at RT for 2h. The mixture was then filtered through a pad of Celite and washed with ethyl acetate (30 mL). Combined filtrates were concentrated to dryness. The resulting solid was triturated in hexanes (20mL) and filtered to give a white solid of compound Ib (230 mg).

## Claims

1. A process for synthesizing a compound of formula 5: 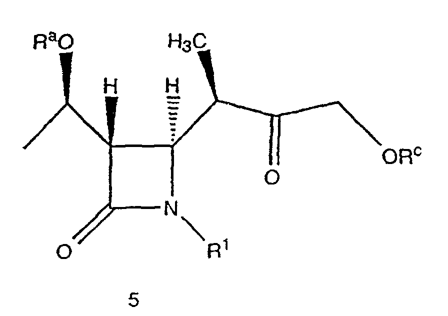 wherein:
R^{a} is:
(1) hydrogen,
(2) C₁₋₄ alkyl or
(3) a hydroxy protecting group; and
R^{b} and R^{c} are independently: tri-organo-silyl, selected from tri-C₁₋₆ alkyl silyl, phenyl di-C₁₋₆ alkyl silyl, and diphenyl mono C₁₋₆ alkyl silyl;
straight and branched lower alkyl
having from 1 to 10 carbon atoms; alkenyl or alkynyl, having from 2 to 10 carbon atoms; cycloalkyl having from 3 to 6 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; aryl, such as phenyl and naphthyl; aralkyl such as benzyl, phenethyl and the like; heterocyclyl (saturated and unsaturated) comprising mono- and bicyclic structures having from 5 to 10 ring atoms, wherein one or more of the heteroatoms is selected from oxygen, nitrogen, or sulfur, such as thiophene, imidazolyl, tetrazolyl,furyl and the like; heterocycloalkyl which comprises the immediately preceding heterocyclyl moieties and the alkyl moiety comprises from 1-10 carbon atoms; substituted species of the above named radicals wherein the substituents are selected from the group consisting of amino, hydroxyl, cyano, carboxyl, nitro, chloro, bromo, or fluoro; lower alkoxy having from 1 to 6 carbon atoms; mercapto; perhaloloweralkyl such as trifluoromethyl; lower alkylthio; guanidino; amidino; sulfamoyl; N-substituted sulfamoyl, amidino, and guanidino wherein the N-substituent is lower alkyl having from 1 to 6 carbon atoms or aryl having 6-10 carbon atoms; COR or CO₂R, wherein R is lower alkyl having from 1 to 6 carbon atoms or aryl having 6-10 carbon atoms; wherein lower alkyl is C₁₋₆ alkyl and wherein R^{b} and R^{c} may be the same or different, but R^{b} must be removable in the presence of R^{c}; and
R¹ is an alkylsilyl protecting group corresponding to the silylating agent employed;
comprising: combining the ketoester of formula 4: 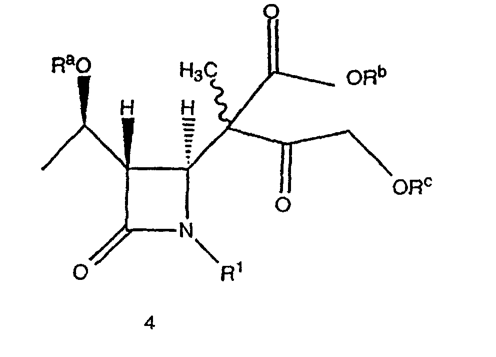 wherein R^{a}, R^{b}, R^{c} and R¹ are described above;
with an acid and a first catalyst at a temperature of 0°C to 50°C with 0-3447.38 kPa (0-500 psi) H₂, to give a compound of formula 5.

2. A process according to claim 1 wherein R^{a} is a hydroxy protecting group and R^{b} and R^{c} are independently selected from the group consisting of benzyl, ethoxycarbonyl, t-butyloxycarbonyl, benzyloxycarbonyl, allyloxycarbonyl, fluoroenylmethyloxycarbonyl, t-butyldimethylsilyl, and isopropyldimethylsilyl.

3. A process according to claim 2 wherein R^{a} is selected from the group consisting of tri-C₁₋₆ alkyl silyl, phenyl di C₁₋₆ alkyl silyl, diphenyl mono C₁₋₆ alkyl silyloxy, *tert*-butyldimethylsilyl, substituted and unsubstituted benzyl, allyl, triethylsilyl, t-butyloxycarbonyl, o-nitrobenzyloxycarbonyl, p-nitrobenzyloxycarbonyl, 2,2,2-trichloroethyloxycarbonyl, benzyloxycarbonyl, allyloxycarbonyl, and fluorenylmethyloxycarbonyl and R^{b} and R^{c} are independently benzyl, substituted benzyl, ethoxycarbonyl, or t-butyloxy carbonyl, wherein said benzyl, when substituted, is substituted by 1 to 3 groups selected from C₁₋₆alkyl, NO₂ and halogen.

4. A process according to claim 1 wherein R^{b} and R^{c} are independently selected from *tert*-butyl-dimethylsilyl, hexyldimethylsilyl and isopropyl-dimethylsilyl.

5. A process according to claim 1 wherein Ra is *tert*-butyldimethylsilyl.

6. A process according to claim 1 wherein the acid is mono-, di-, or tri-carboxylic acid and the first catalyst is Pd/C, Pd(OH)₂/C or (Ra)Ni.

7. A process according to claim 6 wherein the acid is acetic acid or formic acid and the first catalyst is Pd/C or Pd(OH)₂/C.

8. A process according to claim 1 wherein the reaction is carried out at 68.95-689.48 kPa (10-100 psi) H₂.

9. A process according to claim 7 wherein the reaction is carried out at 137.90-275.79 kPa (20-40 psi) H₂.

10. A process for synthesizing a compound of formula 6: 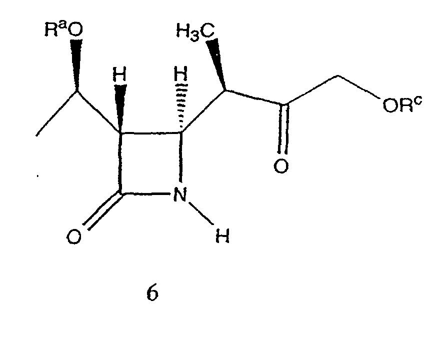 wherein:
R^{a} is:
(1) hydrogen,
(2) C₁₋₄ alkyl or
(3) a hydroxy protecting group; and
R^{b} and R^{c} are independently: tri-organo-silyl, selected from tri-C₁₋₆ alkyl silyl, phenyl di-C₁₋₆ alkyl silyl, and diphenyl mono C₁₋₆ alkyl silyl; straight and branched lower alkyl having from 1 to 10 carbon atoms; alkenyl or alkynyl, having from 2 to 10 carbon atoms; cycloalkyl having from 3 to 6 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; aryl, such as phenyl and naphthyl; aralkyl such as benzyl, phenethyl and the like; heterocyclyl (saturated and unsaturated) comprising mono- and bicyclic structures having from 5 to 10 ring atoms, wherein one or more of the heteroatoms is selected from oxygen, nitrogen, or sulfur, such as thiophene, imidazolyl, tetrazolyl,furyl and the like; heterocycloalkyl which comprises the immediately preceding heterocyclyl moieties and the alkyl moiety comprises from 1-10 carbon atoms; substituted species of the above named radicals wherein the substituents are selected from the group consisting of amino, hydroxyl, cyano, carboxyl, nitro, chloro, bromo, or fluoro; lower alkoxy having from 1 to 6 carbon atoms; mercapto; perhaloloweralkyl such as trifluoromethyl; lower alkylthio; guanidino; amidino; sulfamoyl; N-substituted sulfamoyl, amidino, and guanidino wherein the N-substituent is lower alkyl having from 1 to 6 carbon atoms or aryl having 6-10 carbon atoms; COR or CO₂R, wherein R is lower alkyl having from 1 to 6 carbon atoms or aryl having 6-10 carbon atoms; wherein lower alkyl is C₁₋₆ alkyl and wherein R^{b} and R^{c} may be the same or different, but R^{b} must be removable in the presence of R^{c}; and
comprising: combining the ketoester of formula 4: 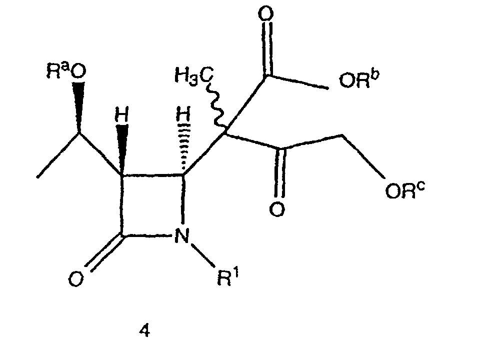 wherein R^{a,} R^{b}, and R^{c} are defined above and R¹ is an alkylsilyl protecting group corresponding to the silylating agent employed;
with an acid and a first catalyst at a temperature of 0°C to 50°C with 0-3447.38 kPa (0-500 psi) H₂,to give a compound of formula 5: 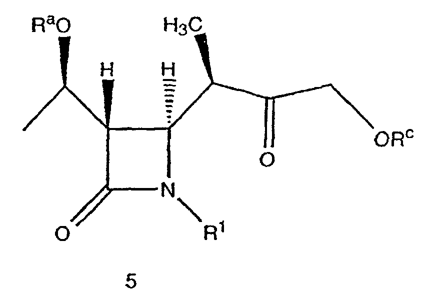 and selectively desilylating with a desilylating agent at a temperature of -10°C to 50°C to yield the compound of formula 6.

11. A process according to claim 10 wherein R^{a} is a hydroxy protecting group and R^{b} and R^{c} are independently selected from the group consisting of benzyl, substituted benzyl, ethoxycarbonyl, t-butyloxycarbonyl, benzyloxycarbonyl, allyloxycarbonyl, fluorenylmethyloxycarbonyl, t-butyldimethylsilyl, and isopropyldimethylsilyl, wherein said benzyl, when substituted, is substituted by 1 to 3 groups selected from C₁₋₆alkyl, NO₂ and halogen.

12. A process according to claim 11 wherein R^{a} is selected from the group consisting of tri-C₁₋₆ alkyl silyl, phenyl di C₁₋₆ alkyl silyl, diphenyl mono C₁₋₆ alkyl silyloxy, *tert*-butyldimethylsilyl, substituted and unsubstituted benzyl, allyl, triethylsilyl, t-butyloxycarbonyl, o-nitrobenzyloxycarbonyl, p-nitrobenzyloxycarbonyl, 2,2,2-trichloroethyloxycarbonyl, benzyloxycarbonyl, allyloxycarbonyl, and fluorenylmethyloxycarbonyl and R^{b} and R^{c} are independently benzyl, substituted benzyl, ethoxycarbonyl, or t-butyloxy carbonyl, wherein said benzyl, when substituted, is substituted by 1 to 3 groups selected from C₁₋₆alkyl, NO₂ and halogen.

13. A process according to claim 10 wherein R^{b} and R^{c} are independently selected from *tert*-butyl dimethylsilyl, hexyldimethylsilyl and ispropyl-dimethylsilyl.

14. A process according to claim 10 wherein Ra is *tert*-butyldimethylsilyl.

15. A process according to claim 10 wherein the desilylating agent is selected from the group consisting of NaOH, KOH, the acid is mono-, di-, or tri-carboxylic acid and N-desilylating agent and the first catalyst is Pd/C, Pd(OH)₂/C or (Ra)Ni..

16. A process according to claim 15 wherein the desilylating agent is an N-desilylating agent selected from the group consisting of tetrabutylammoniumfluoride (TBAF) or 2 mercaptopyridine N-oxide, the acid is acetic acid or formic acid and the first catalyst is Pd/C or Pd(OH₂)/C.

17. A process according to claim 10 wherein the reaction is carried out at 68.95-689.48 kPa (10-100 psi) H₂.

18. A process according to claim 15 wherein the reaction is carried out at 137.90-275.79 kPa (20-40 psi) H₂.

19. A process for synthesizing a compound of formula I: 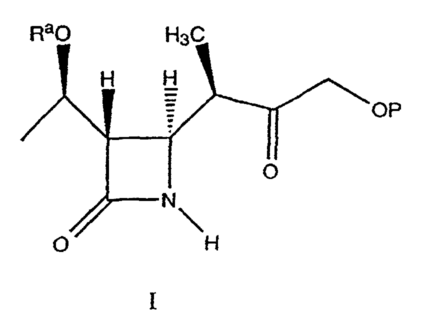 wherein:
R^{a} and P are independently:
(1) hydrogen,
(2) C₁₋₄ alkyl or
(3) a hydroxy protecting group; and
comprising:
(a) reacting a ketoester of formula 1: 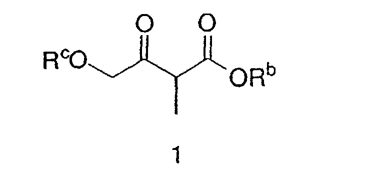
R^{b} and R^{c} are independently: H, tri-organo-silyl, selected from tri-C₁₋₆ alkyl silyl, phenyl di-C₁₋₆ alkyl silyl, and diphenyl mono C₁₋₆ alkyl silyl; straight and branched lower alkyl
having from 1 to 10 carbon atoms; alkenyl or alkynyl, having from 2 to 10 carbon atoms; cycloalkyl having from 3 to 6 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; aryl, such as phenyl and naphthyl; aralkyl such as benzyl, phenethyl and the like; heterocyclyl (saturated and unsaturated) comprising mono- and bicyclic structures having from 5 to 10 ring atoms, wherein one or more of the heteroatoms is selected from oxygen, nitrogen, or sulfur, such as thiophene, imidazolyl, tetrazolyl, furyl and the like; heterocycloalkyl which comprises the immediately preceding heterocyclyl moieties and the alkyl moiety comprises from 1-10 carbon atoms; substituted species of the above named radicals wherein the substituents are selected from the group consisting of amino, hydroxyl, cyano, carboxyl, nitro, chloro, bromo, or fluoro; lower alkoxy having from 1 to 6 carbon atoms; mercapto; perhaloloweralkyl such as trifluoromethyl; lower alkylthio; guanidino; amidino; sulfamoyl; N-substituted sulfamoyl, amidino, and guanidino wherein the N-substituent is lower alkyl having from 1 to 6 carbon atoms or aryl having 6-10 carbon atoms; COR or CO₂R, wherein R is lower alkyl having from 1 to 6 carbon atoms or aryl having 6-10 carbon atoms; wherein lower alkyl is C₁₋₆ alkyl and wherein R^{b} and R^{c} may be the same or different, but R^{b} must be removable in the presence of R^{c};
with a compound of formula 2: 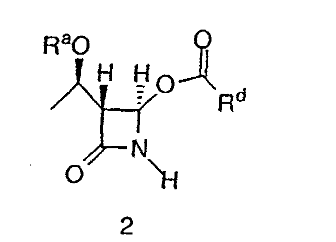 wherein R^{a} is defined above, and R^{d} is C₁₋₄ alkyl;
in the presence of a first base at a temperature of 25°C to 60° C to produce a ketoester adduct of formula 3: 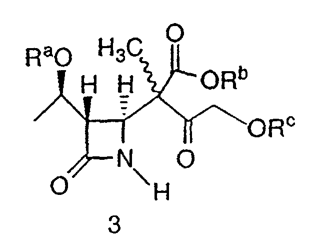
(b) combining the compound of formula 3 with a silylating agent and a second base to produce the ketoester of formula 4: 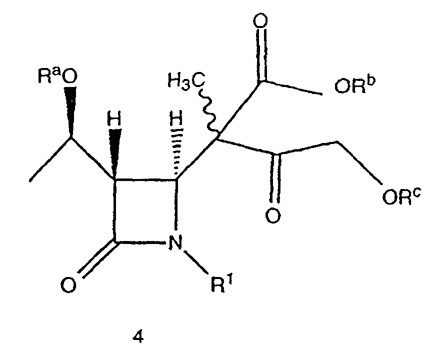 wherein R^{a}, R^{b}, and R^{c} are defined above and R¹ is an alkylsilyl protecting group corresponding to the silylating agent employed;
(c) combining the isolated ketoester of formula 4 with an acid and a first catalyst at a temperature of 0°C to 50°C with 0-3447.38 kPa (0-500 psi) H₂, to give a compound of formula 5: 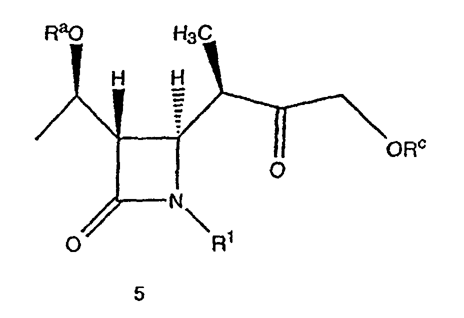
(d) selectively desilylating with a desilylating agent at a temperature of -10°C to 50°C to yield the compound of formula 6: 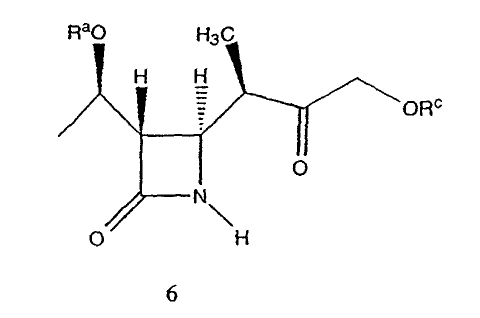
(e) dissolving the compound of formula 6 in an alcholol and hydrogenating at 206.84 to 379.21 kPa (30 to 55 psi) H2,in the presence of a second catalyst at a temperature of 0°C to 100°C, to yield compound I and
(f) purifying and isolating compound I.

20. A process according to claim 19 wherein R^{a} is a hydroxy protecting group and R^{b} and R^{c} are independently selected from the group consisting of benzyl, substituted benzyl, ethoxycarbonyl, t-butyloxycarbonyl, benzyloxycarbonyl, allyloxycarbonyl, fluorenylmethyloxycarbonyl, t-butyldimethylsilyl, and isopropyldimethylsilyl, wherein said benzyl, when substituted, is substituted by 1 to 3 groups selected from C₁₋₆alkyl, NO₂ and halogen.

21. A process according to claim 20 wherein R^{a} is selected from the group consisting of tri-C₁₋₆ alkyl silyl, phenyl di C₁₋₆ alkyl silyl, diphenyl mono C₁₋₆ alkyl silyloxy, *tert*-butyldimethylsilyl, substituted and unsubstituted benzyl, allyl, triethylsilyl, t-butyloxycarbonyl, o-nitrobenzyloxycarbonyl, p-nitrobenzyloxycarbonyl, 2,2,2-trichloroethyloxycarbonyl, benzyloxycarbonyl, allyloxycarbonyl, and fluorenylmethyloxycarbonyl and R^{b} and R^{c} are independently H, benzyl, substituted benzyl, ethoxycarbonyl, or t-butyloxy carbonyl, wherein said benzyl, when substituted, is substituted by 1 to 3 groups selected from C₁₋₆alkyl, NO₂ and halogen.

22. A process according to claim 19 wherein R^{b} and R^{c} are independently selected from tert-butyl-dimethylsilyl, hexyldimethylsilyl and isopropyl-dimethylsilyl.

23. A process according to claim 19 wherein Ra is *tert*-butyldimethylsilyl.

24. A process according to claim 19 wherein the reaction at step (c) is carried out at 68.95-689.48 kPa (10-100 psi) H₂.

25. A process according to claim 24 wherein the reaction at step (c) is carried out at 137.90-275.79 kPa (20-40 psi) H₂.

26. A process according to claim 19 wherein the first and second bases are selected from the group consisting of potassium carbonate, calcium carbonate, bicarbonate, diisopropylethylamine, triethylamine, dimethylethylamine, dimethylpentylamine and diazabicycloundecane; the silylating agent is selected from the group consisting of trialkylsilylchlorides, trialkylsilyliodides and triflates and the desilylating agent is selected from the group consisting of NaOH, KOH, and N-desilylating agent.

27. A process according to claim 26 wherein the first base is potassium carbonate or diazabicycloundecane, the second base is triethylamine or diazabicycloundecane; the silylating agent is trimethylsilyltriflate (TMSOTf), tert-butyl dimethylsilyltriflate (TBSOTf), triethylsilyltriflate (TESOTf) or tert-butyl dimethylsilyl chloride/sodium iodide (TBSCl/NaI); and the desilylating agent is an N-desilylating agent selected from the group consisting of tetrabutylammoniumfluoride (TBAF) or 2-mercaptopyridine N-oxide.

28. A process according to claim 19 wherein the acid is mono-, di-, or tri-carboxylic acid; the alcohol is methanol, ethanol, butanol, pentanol, 1-propanol or 2-propanol and the first and second catalyst are Pd/C, Pd(OH)₂/C or (Ra)Ni.

29. A process according to claim 28 wherein the acid is acetic acid or formic acid; the alcohol is methanol or ethanol and the first and second catalyst are Pd/C or Pd(OH)₂/C.

## Patentansprüche

1. Ein Verfahren zur Synthese einer Verbindung der Formel 5: 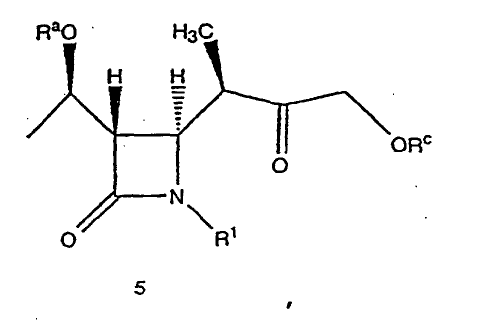 wobei:
R^{a} ist:
(1) Wasserstoff,
(2) C₁₋₄-Alkyl oder
(3) eine Hydroxy-Schutzgruppe, und
R^{b} und R^{c} unabhängig sind: Triorganosilyl, ausgewählt aus Tri-C₁₋₆-alkylsilyl, Phenyldi-C₁₋₆-alkylsilyl und Diphenylmono-C₁₋₆-alkylsilyl; gerades und verzweigtes Niedrigalkyl mit 1 bis 10 Kohlenstoffatomen; Alkenyl oder Alkinyl mit 2 bis 10 Kohlenstoffatomen; Cycloalkyl mit 3 bis 6 Kohlenstoffatomen; Cycloalkylalkyl, wobei der Cycloalkylrest 3 bis 6 Kohlenstoffatome umfaßt und der Alkylrest 1 bis 10 Kohlenstoffatome umfaßt; Alkylcycloalkyl, wobei der Alkylrest 1 bis 6 Kohlenstoffatome umfaßt und der Cyclo-alkylrest 3 bis 6 Kohlenstoffatome umfaßt; Aryl, wie z.B. Phenyl und Naphthyl; Aralkyl, wie z.B. Benzyl, Phenethyl und dergleichen; Heterocyclyl (gesättigt und ungesättigt), das mono- und bicyclische Strukturen mit 5 bis 10 Ringatomen umfaßt, wobei eines oder mehrere der Heteroatome ausgewählt ist/sind aus Sauerstoff, Stick-stoff oder Schwefel, wie z.B. Thiophen, Imidazolyl, Tetrazolyl, Furyl und dergleichen; Heterocycloalkyl, das die unmittelbar vorausgehenden Heterocyclylreste umfaßt, und wobei der Alkylrest 1-10 Kohlenstoffatome umfaßt; substituierte Spezies der oben genannten Reste, wobei die Substituenten ausgewählt sind aus der Gruppe, bestehend aus Amino, Hydroxyl, Cyano, Carboxyl, Nitro, Chlor, Brom oder Fluor; Niedrigalkoxy mit 1 bis 6 Kohlenstoffatomen; Mercapto; Perhalogenniedrigalkyl, wie z.B. Trifluormethyl; Niedrigalkylthio; Guanidino; Amidino; Sulfamoyl; N-substituiertes Sulfamoyl, Amidino und Guanidino, wobei der N-Substituent Niedrigalkyl mit 1 bis 6 Kohlenstoffatomen oder Aryl mit 6-10 Kohlenstoff-atomen ist; COR oder CO₂R, wobei R Niedrigalkyl mit 1 bis 6 Kohlenstoffatomen oder Aryl mit 6-10 Kohlenstoffatomen ist; wobei Niedrigalkyl C₁₋₆-Alkyl ist und wobei R^{b} und R^{c} gleich oder verschieden sein können, R^{b} jedoch in Gegenwart von R^{c} entfernbar sein muß; und
R¹ eine Alkylsilyl-Schutzgruppe ist, die dem eingesetzten Silylierungsmittel entspricht,
umfassend: Vereinen des Ketoesters der Formel 4: 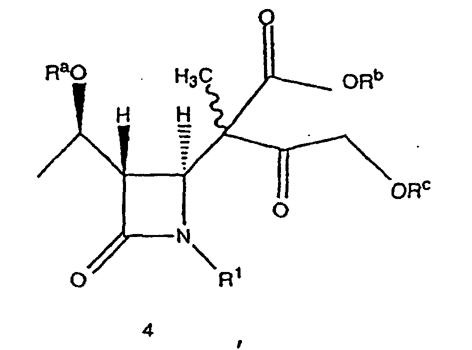 wobei R^{a}, R^{b}, R^{c} und R¹ wie oben beschrieben sind,
mit einer Säure und einem ersten Katalysator bei einer Temperatur von 0°C bis 50°C mit 0-3447,38 kPa (0-500 psi) H₂, um eine Verbindung der Formel 5 zu ergeben.

2. Ein Verfahren gemäß Anspruch 1, wobei R^{a} eine Hydroxy-Schutzgruppe ist und R^{b} und R^{c} unabhängig ausgewählt sind aus der Gruppe, bestehend aus Benzyl, Ethoxycarbonyl, t-Butyloxycarbonyl, Benzyloxycarbonyl, Allyloxycarbonyl, Fluorenylmethyloxycarbonyl, t-Butyldimethylsilyl und Isopropyldimethylsilyl.

3. Ein Verfahren gemäß Anspruch 2, wobei R^{a} ausgewählt ist aus der Gruppe, bestehend aus Tri-C₁₋₆-alkylsilyl, Phenyldi-C₁₋₆-alkylsilyl, Diphenylmono-C₁₋₆-alkylsilyloxy, tert.-Butyldimethylsilyl, substituiertem und unsubstituiertem Benzyl, Allyl, Triethylsilyl, t-Butyloxycarbonyl, o-Nitrobenzyloxycarbonyl, p-Nitrobenzyloxycarbonyl, 2,2,2-Trichlorethyloxycarbonyl, Benzyloxycarbonyl, Allyloxycarbonyl und Fluorenylmethyloxycarbonyl, und R^{b} und R^{c} unabhängig Benzyl, substituiertes Benzyl, Ethoxycarbonyl oder t-Butyloxycarbonyl sind, wobei das Benzyl, wenn es substituiert ist, durch 1 bis 3 Gruppen, ausgewählt aus C₁₋₆-Alkyl, NO₂ und Halogen, substituiert ist.

4. Ein Verfahren gemäß Anspruch 1, wobei R^{b} und R^{c} unabhängig ausgewählt sind aus tert.-Butyldimethylsilyl, Hexyldimethylsilyl und Isopropyldimethylsilyl.

5. Ein Verfahren gemäß Anspruch 1, wobei Ra tert.-Butyldimethyisilyl ist.

6. Ein Verfahren gemäß Anspruch 1, wobei die Säure Mono-, Dioder Tricarbonsäure ist und der erste Katalysator Pd/C, Pd(OH)₂/C oder (Ra)Ni ist.

7. Ein Verfahren gemäß Anspruch 6, wobei die Säure Essigsäure oder Ameisensäure ist und der erste Katalysator Pd/C oder Pd(OH)₂/C ist.

8. Ein Verfahren gemäß Anspruch 1, wobei die Reaktion bei 68,95-689,48 kPa (10-100 psi) H₂ durchgeführt wird.

9. Ein Verfahren gemäß Anspruch 7, wobei die Reaktion bei 137,90-275,79 kPa (20-40 psi) H₂ durchgeführt wird.

10. Ein Verfahren zur Synthese einer Verbindung der Formel 6: 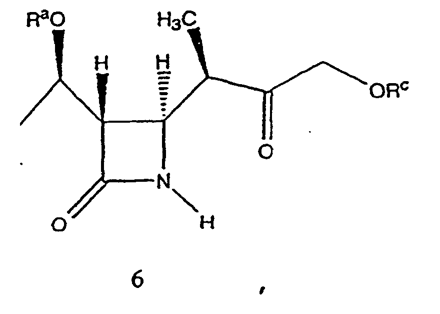 wobei:
R^{a} ist:
(1) Wasserstoff,
(2) C₁₋₄-Alkyl oder
(3) eine Hydroxy-Schutzgruppe, und
R^{b} und R^{c} unabhängig sind: Triorganosilyl, ausgewählt aus Tri-C₁₋₆-alkylsilyl, Phenyldi-C₁₋₆-alkylsilyl und Diphenylmono-C₁₋₆-alkylsilyl; gerades und verzweigtes Niedrigalkyl mit 1 bis 10 Kohlenstoffatomen; Alkenyl oder Alkinyl mit 2 bis 10 Kohlenstoffatomen; Cycloalkyl mit 3 bis 6 Kohlenstoffatomen; Cycloalkylalkyl, wobei der Cycloalkylrest 3 bis 6 Kohlenstoffatome umfaßt und der Alkylrest 1 bis 10 Kohlenstoffatome umfaßt; Alkylcycloalkyl, wobei der Alkylrest 1 bis 6 Kohlenstoffatome umfaßt und der Cycloalkylrest 3 bis 6 Kohlenstoffatome umfaßt; Aryl, wie z.B. Phenyl und Naphthyl; Aralkyl, wie z.B. Benzyl, Phenethyl und dergleichen; Heterocyclyl (gesättigt und ungesättigt), das mono- und bicyclische Strukturen mit 5 bis 10 Ringatomen umfaßt, wobei eines oder mehrere der Heteroatome ausgewählt ist/sind aus Sauerstoff, Stickstoff oder Schwefel, wie z.B. Thiophen, Imidazolyl, Tetrazolyl, Furyl und dergleichen; Heterocycloalkyl, das die unmittelbar vorausgehenden Heterocyclylreste umfaßt, und wobei der Alkylrest 1-10 Kohlenstoffatome umfaßt; substituierte Spezies der oben genannten Reste, wobei die Substituenten ausgewählt sind aus der Gruppe, bestehend aus Amino, Hydroxyl, Cyano, Carboxyl, Nitro, Chlor, Brom oder Fluor; Niedrigalkoxy mit 1 bis 6 Kohlenstoffatomen; Mercapto; Perhalogenniedrigalkyl, wie z.B. Trifluormethyl; Niedrigalkylthio; Guanidino; Amidino; Sulfamoyl; N-substituiertes Sulfamoyl, Amidino und Guanidino, wobei der N-Substituent Niedrigalkyl mit 1 bis 6 Kohlenstoffatomen oder Aryl mit 6-10 Kohlenstoffatomen ist; COR oder CO₂R, wobei R Niedrigalkyl mit 1 bis 6 Kohlenstoffatomen oder Aryl mit 6-10 Kohlenstoffatomen ist; wobei Niedrigalkyl C₁₋₆-Alkyl ist und wobei R^{b} und R^{c} gleich oder verschieden sein können, R^{b} jedoch in Gegenwart von R^{c} entfernbar sein muß; und
umfassend: Vereinen des Ketoesters der Formel 4: 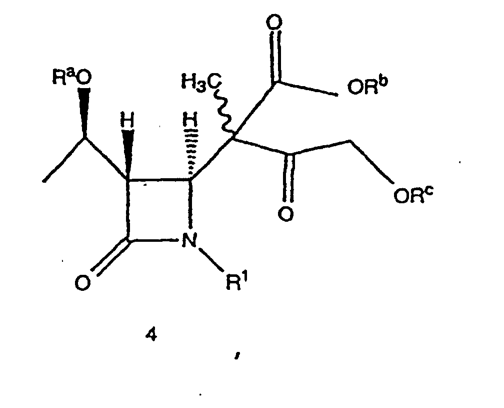 wobei R^{a}, R^{b} und R^{c} wie oben definiert sind und R¹ eine Alkylsilyl-Schutzgruppe ist, die dem eingesetzten Silylierungsmittel entspricht,
mit einer Säure und einem ersten Katalysator bei einer Temperatur von 0°C bis 50°C mit 0-3447,38 kPa (0-500 psi) H₂, um eine Verbindung der Formel 5 zu ergeben: 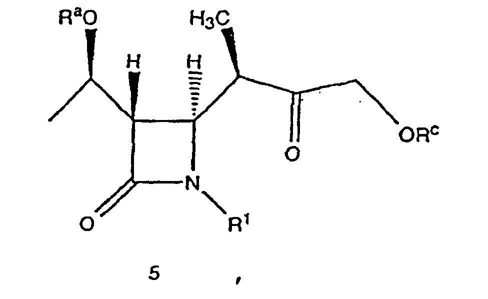 und selektive Desilylierung mit einem Desilylierungsmittel bei einer Temperatur von -10°C bis 50°C, um die Verbindung der Formel 6 zu ergeben.

11. Ein Verfahren gemäß Anspruch 10, wobei R^{a} eine Hydroxy-Schutzgruppe ist und R^{b} und R^{c} unabhängig ausgewählt sind aus der Gruppe, bestehend aus Benzyl, substituiertem Benzyl, Ethoxycarbonyl, t-Butyloxycarbonyl, Benzyloxycarbonyl, Allyloxycarbonyl, Fluorenylmethyloxycarbonyl, t-Butyldimethylsilyl und Isopropyldimethylsilyl, wobei das Benzyl, wenn es substituiert ist, durch 1 bis 3 Gruppen, ausgewählt aus C₁₋₆-Alkyl, NO₂ und Halogen, substituiert ist.

12. Ein Verfahren gemäß Anspruch 11, wobei R^{a} ausgewählt ist aus der Gruppe, bestehend aus Tri-C₁₋₆-alkylsilyl, Phenyldi-C₁₋₆alkylsilyl, Diphenylmono-C₁₋₆-alkylsilyloxy, tert.-Butyldimethylsilyl, substituiertem und unsubstituiertem Benzyl, Allyl, Triethylsilyl, t-Butyloxycarbonyl, o-Nitrobenzyloxycarbonyl, p-Nitrobenzyloxycarbonyl, 2,2,2-Trichlorethyloxycarbonyl, Benzyloxycarbonyl, Allyloxycarbonyl und Fluorenylmethyloxycarbonyl, und R^{b} und R^{c} unabhängig Benzyl, substituiertes Benzyl, Ethoxycarbonyl oder t-Butyloxycarbonyl sind, wobei das Benzyl, wenn es substituiert ist, durch 1 bis 3 Gruppen, ausgewählt aus C₁₋₆-Alkyl, NO₂ und Halogen, substituiert ist.

13. Ein Verfahren gemäß Anspruch 10, wobei R^{b} und R^{c} unabhängig ausgewählt sind aus tert.-Butyldimethylsilyl, Hexyldimethylsilyl und Isopropyldimethylsilyl.

14. Ein Verfahren gemäß Anspruch 10, wobei Ra tert.-Butyldimethylsilyl ist.

15. Ein Verfahren gemäß Anspruch 10, wobei das Desilylierungsmittel ausgewählt ist aus der Gruppe, bestehend aus NaOH, KOH, die Säure Mono-, Di- oder Tricarbonsäure und N-Desilylierungsmittel ist und der erste Katalysator Pd/C, Pd(OH)₂/C oder (Ra)Ni ist.

16. Ein Verfahren gemäß Anspruch 15, wobei das Desilylierungsmittel ein N-Desilylierungsmittel ist, ausgewählt aus der Gruppe, bestehend aus Tetrabutylammoniumfluorid (TBAF) oder 2-Mercaptopyridin-N-oxid, die Säure Essigsäure oder Ameisensäure ist und der erste Katalysator Pd/C oder Pd(OH)₂/C ist.

17. Ein Verfahren gemäß Anspruch 10, wobei die Reaktion bei 68,95-689,48 kPa (10-100 psi) H₂ durchgeführt wird.

18. Ein Verfahren gemäß Anspruch 15, wobei die Reaktion bei 137,90-275,79 kPa (20-40 psi) H₂ durchgeführt wird.

19. Ein Verfahren zur Synthese einer Verbindung der Formel I: 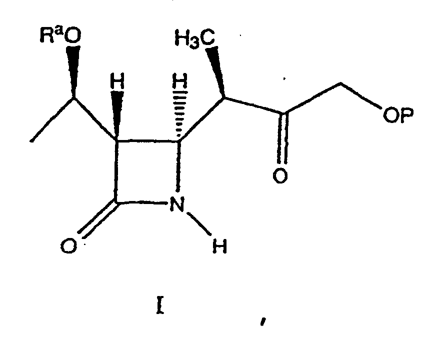 wobei:
R^{a} und P unabhängig sind:
(1) Wasserstoff,
(2) C₁₋₄-Alkyl oder
(3) eine Hydroxy-Schutzgruppe, und
umfassend:
(a) Umsetzung eines Ketoesters der Formel 1: 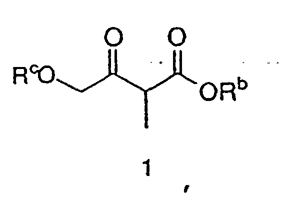 wobei
R^{b} und R^{c} unabhängig sind: H, Triorganosilyl, ausgewählt aus Tri-C₁₋₆-alkylsilyl, Phenyldi-C₁₋₆-alkylsilyl und Diphenylmono-C₁₋₆-alkylsilyl; gerades und verzweigtes Niedrigalkyl mit 1 bis 10 Kohlenstoffatomen; Alkenyl oder Alkinyl mit 2 bis 10 Kohlenstoffatomen; Cycloalkyl mit 3 bis 6 Kohlenstoffatomen; Cycloalkylalkyl, wobei der Cycloalkylrest 3 bis 6 Kohlenstoffatome umfaßt und der Alkylrest 1 bis 10 Kohlenstoffatome umfaßt; Alkylcycloalkyl, wobei der Alkylrest 1 bis 6 Kohlenstoffatome umfaßt und der Cycloalkylrest 3 bis 6 Kohlenstoffatome umfaßt; Aryl, wie z.B. Phenyl und Naphthyl; Aralkyl, wie z.B. Benzyl, Phenethyl und dergleichen; Heterocyclyl (gesättigt und ungesättigt), das mono- und bicyclische Strukturen mit 5 bis 10 Ringatomen umfaßt, wobei eines oder mehrere der Heteroatome ausgewählt ist/sind aus Sauerstoff, Stickstoff oder Schwefel, wie z.B. Thiophen, Imidazolyl, Tetrazolyl, Furyl und dergleichen; Heterocycloalkyl, das die unmittelbar vorausgehenden Heterocyclylreste umfaßt, und wobei der-Alkylrest 1-10 Kohlenstoffatome umfaßt; substituierte Spezies der oben genannten Reste, wobei die Substituenten ausgewählt sind aus der Gruppe, bestehend aus Amino, Hydroxyl, Cyano, Carboxyl, Nitro, Chlor, Brom oder Fluor; Niedrigalkoxy mit 1 bis 6 Kohlenstoffatomen; Mercapto; Perhalogenniedrigalkyl, wie z.B. Trifluormethyl; Niedrigalkylthio; Guanidino; Amidino; Sulfamoyl; N-substituiertes Sulfamoyl, Amidino und Guanidino, wobei der N-Substituent Niedrigalkyl mit 1 bis 6 Kohlenstoffatomen oder Aryl mit 6-10 Kohlenstoffatomen ist; COR oder CO₂R, wobei R Niedrigalkyl mit 1 bis 6 Kohlenstoffatomen oder Aryl mit 6-10 Kohlenstoffatomen ist; wobei Niedrigalkyl C₁₋₆-Alkyl ist und wobei R^{b} und R^{c} gleich oder verschieden sein können, R^{b} jedoch in Gegenwart von R^{c} entfernbar sein muß;
mit einer Verbindung der Formel 2: 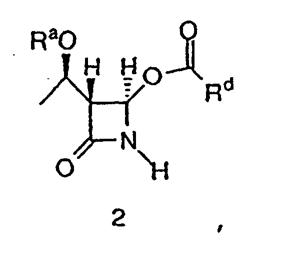 wobei R^{a} oben definiert ist und R^{d} C₁₋₄-Alkyl ist,
in Gegenwart einer ersten Base bei einer Temperatur von 25°C bis 60°C, um ein Ketoester-Addukt der Formel 3 zu erzeugen: 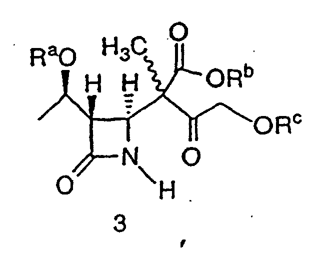
(b) Vereinen der Verbindung der Formel 3 mit einem Silylierungsmittel und einer zweiten Base, um den Ketoester der Formel 4 zu erzeugen: 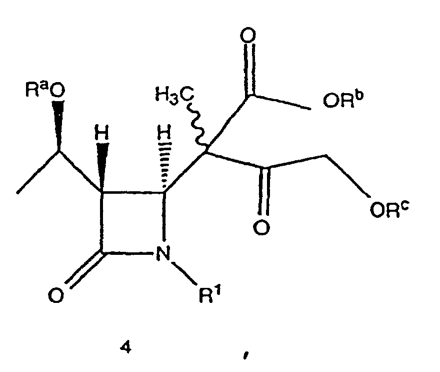 wobei R^{a}, R^{b} und R^{c} oben definiert sind und R¹ eine Alkylsilyl-Schutzgruppe ist, die dem eingesetzten Silylierungsmittel entspricht,
(c) Vereinen des isolierten Ketoesters der Formel 4 mit einer Säure und einem ersten Katalysator bei einer Temperatur von 0°C bis 50°C mit 0-3447,38 kPa (0-500 psi) H₂, um eine Verbindung der Formel 5 zu ergeben: 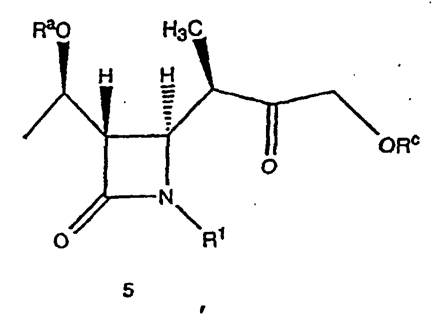
(d) selektive Desilylierung mit einem Desilylierungsmittel bei einer Temperatur von -10°C bis 50°C, um die Verbindung der Formel 6 zu ergeben: 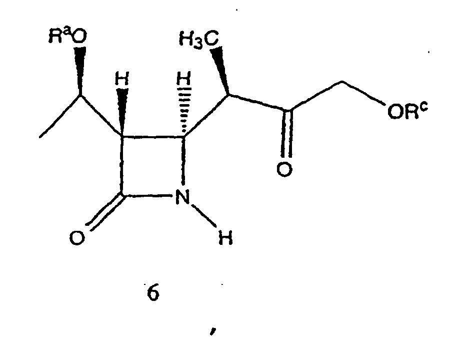
(e) Lösen der Verbindung der Formel 6 in einem Alkohol und Hydrieren bei 206,84 bis 379,21 kPa (30 bis 55 psi) H₂ in Gegenwart eines zweiten Katalysators bei einer Temperatur von 0°C bis 100°C, um Verbindung I zu ergeben, und
(f) Reinigung und Isolierung von Verbindung I.

20. Ein Verfahren gemäß Anspruch 19, wobei R^{a} eine Hydroxy-Schutzgruppe ist und R^{b} und R^{c} unabhängig ausgewählt sind aus der Gruppe, bestehend aus Benzyl, substituiertem Benzyl, Ethoxycarbonyl, t-Butyloxycarbonyl, Benzyloxycarbonyl, Allyloxycarbonyl, Fluorenylmethyloxycarbonyl, t-Butyldimethylsilyl und Isopropyldimethylsilyl, wobei das Benzyl, wenn es substituiert ist, durch 1 bis 3 Gruppen, ausgewählt aus C₁₋₆-Alkyl, NO₂ und Halogen, substituiert ist.

21. Ein Verfahren gemäß Anspruch 20, wobei R^{a} ausgewählt ist aus der Gruppe, bestehend aus Tri-C₁₋₆-alkylsilyl, Phenyldi-C₁₋₆alkylsilyl, Diphenylmono-C₁₋₆-alkylsilyloxy, tert.-Butyldimethylsilyl, substituiertem und unsubstituiertem Benzyl, Allyl, Triethylsilyl, t-Butyloxycarbonyl, o-Nitrobenzyloxycarbonyl, p-Mitrobenzyloxycarbonyl, 2,2,2-Trichlorethyloxycarbonyl, Benzyloxycarbonyl, Allyloxycarbonyl und Fluorenylmethyloxycarbonyl, und R^{b} und R^{c} unabhängig H, Benzyl, substituiertes Benzyl, Ethoxycarbonyl oder t-Butyloxycarbonyl sind, wobei das Benzyl, wenn es substituiert ist, durch 1 bis 3 Gruppen, ausgewählt aus C₁₋₆-Alkyl, NO₂ und Halogen, substituiert ist.

22. Ein Verfahren gemäß Anspruch 19, wobei R^{b} und R^{c} unabhängig ausgewählt sind aus tert.-Butyldimethylsilyl, Hexyldimethylsilyl und Isopropyldimethylsilyl.

23. Ein Verfahren gemäß Anspruch 19, wobei Ra tert.-Butyldimethylsilyl ist.

24. Ein Verfahren gemäß Anspruch 19, wobei die Reaktion in Schritt (c) bei 68,95-689,48 kPa (10-100 psi) H₂ durchgeführt wird.

25. Ein Verfahren gemäß Anspruch 24, wobei die Reaktion in Schritt (c) bei 137,90-275,79 kPa (20-40 psi) H₂ durchgeführt wird.

26. Ein Verfahren gemäß Anspruch 19, wobei die erste und die zweite Base ausgewählt sind aus der Gruppe, bestehend aus Kaliumcarbonat, Calciumcarbonat, Hydrogencarbonat, Diisopropylethylamin, Triethylamin, Dimethylethylamin, Dimethylpentylamin und Diazabicycloundecan, das Silylierungsmittel ausgewählt ist aus der Gruppe, bestehend aus Trialkylsilylchloriden, Trialkylsilyliodiden und Triflaten, und das Desilylierungsmittel ausgewählt ist aus der Gruppe, bestehend aus NaOH, KOH und N-Desilylierungsmittel.

27. Ein Verfahren gemäß Anspruch 26, wobei die erste Base Kaliumcarbonat oder Diazabicycloundecan ist, die zweite Base Triethylamin oder Diazabicycloundecan ist, das Silylierungsmittel Trimethylsilyltriflat (TMSOTf), tert.-Butyldimethylsilyltriflat (TBSOTf), Triethylsilyltriflat (TESOTf) oder tert.-Butyldimethylsilylchlorid/Natriumiodid (TBSCl/N ist und das Desilylierungsmittel ein N-Desilylierungsmittel ist, ausgewählt aus der Gruppe, bestehend aus Tetrabutylammoniumfluorid (TBAF) oder 2-Mercaptopyridin-N-oxid.

28. Ein Verfahren gemäß Anspruch 19, wobei die Säure Mono-, Di- oder Tricarbonsäure ist, der Alkohol Methanol, Ethanol, Butanol, Pentanol, 1-Propanol oder 2-Propanol ist und der erste und der zweite Katalysator Pd/C, Pd(OH)₂/C oder (Ra)Ni sind.

29. Ein Verfahren gemäß Anspruch 28, wobei die Säure Essigsäure oder Ameisensäure ist, der Alkohol Methanol oder Ethanol ist und der erste und der zweite Katalysator Pd/C oder Pd(OH)₂/C sind.

## Revendications

1. Procédé pour la synthèse d'un composé de formule 5 : 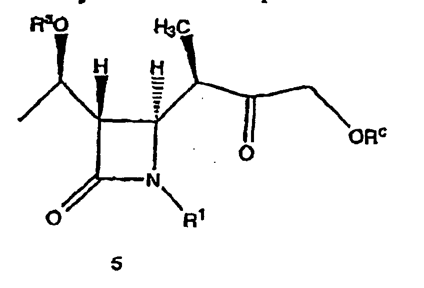 où:
R^{a} est
(1) un hydrogène,
(2) un alkyle en C₁₋₄ ou
(3) un groupe protecteur d'hydroxy ; et
R^{b} et R^{c} sont, de façon indépendante : un reste tri-organo-silyle choisi parmi tri(alkyl en C₁-C₆)silyle, phényldi(alkyl en C₁-C₆)silyle et diphénylmono(alkyl en C₁-C₆)silyle ; un alkyle inférieur, linéaire et ramifié, ayant 1 à 10 atomes de carbone ; un alcényle ou un alcynyle ayant 2 à 10 atomes de carbone; un cycloalkyle ayant 3 à 6 atomes de carbone ; un cycloalkylalkyle dans lequel le fragment cycloalkyle contient 3 à 6 atomes de carbone et le fragment alkyle contient 1 à 10 atomes de carbone ; un alkylcycloalkyle dans lequel le fragment alkyle contient 1 à 10 atomes de carbone et le fragment cycloalkyle contient 3 à 6 atomes de carbone ; un aryle, tel que phényle et naphtyle; un aralkyle tel que benzyle, phénéthyle et analogue; un hétérocyclyle (saturé et insaturé) comprenant des structures mono- et bicycliques ayant 5 à 10 atomes dans le squelette cyclique, un ou plusieurs des hétéroatomes étant choisis parmi l'oxygène, l'azote ou le soufre, tel que thiophène, imidazolyle, tétrazolyle, furyle et analogue; un hétérocycloalkyle comprenant les fragments hétérocyclyle immédiatement ci-dessus et un fragment alkyle comprenant 1 à 10 atomes de carbone ; les espèces substituées dérivées des restes cités ci-dessus dans lesquelles les substituants sont choisis dans le groupe constitué par amino, hydroxy, cyano, carboxyle, nitro, chloro, bromo ou fluoro ; un alcoxy inférieur ayant 1 à 6 atomes de carbone; mercapto; un perhalogénoalkyle inférieur tel que trifluorométhyle ; un alkylthio inférieur ; guanidino; amidino; sulfamoyle; les sulfamoyle, amidino et guanidino N-substitués dans lesquels le N-substituant est un alkyle inférieur ayant 1 à 6 atomes de carbone ou un aryle ayant 6-10 atomes de carbone ; COR ou CO₂R, où R est un alkyle inférieur ayant 1 à 6 atomes de carbone ou un aryle ayant 6-10 atomes de carbone ; où alkyle inférieur est un alkyle en C₁₋₆ et où R^{b} et R^{c} peuvent être identiques ou différents, mais où R^{b} doit obligatoirement pouvoir être éliminé en présence de R^{c} ; et
R¹ est un groupe protecteur alkylsilyle correspondant à l'agent silylant employé ;
comprenant : la combinaison du cétoester de formule 4 : 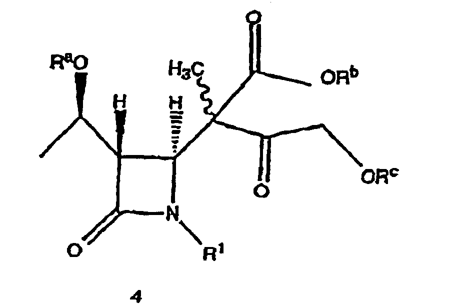 où R^{a}, R^{b}, R^{c} et R¹ sont définis ci-dessus ;
avec un acide et un premier catalyseur à une température de 0°C à 50°C sous une pression de 0 à 3447,38 kPa (0-500 psi) de H₂, pour obtenir un composé de formule 5.

2. Procédé selon la revendication 1, dans lequel R^{a} est un groupe protecteur d'hydroxy et R^{b} et R^{c} sont, de façon indépendante, choisis dans le groupe constitué par benzyle, éthoxycarbonyle, t-bulyloxycarbonyle, benzyloxycarbonyle, allyloxycarbonyle, fluorénylméthyloxycarbonyle, t-butyldiméthylsilyle et isopropyldiméthylsilyle.

3. Procédé selon la revendication 2, dans lequel R^{a} est choisi dans le groupe constitué par tri(alkyl en C₁-C₆)silyle, phényldi(alkyl en C₁-C₆)silyle, diphénylmono(alkyl en C₁-C₆)silyle ; tert-butyldunéthylsilyle, benzyle substitué et non-substitué, allyle, triéthylsilyle, t-butyloxycarbonyle, o-nitrobenzyloxycarbonyle, p-nitrobenzyloxycarbonyle, 2,2,2-trichloro-éthyloxycarbonyle, benzyloxycarbonyle, allyloxycarbonyle et fluorénylméthyloxycarbonyle et R^{b} et R^{c} sont, de façon indépendante, benzyle, benzyle substitué, éthoxycarbonyle ou t-butyloxycarbonyle, où ledit benzyle, quand il est substitué, est substitué par 1 à 3 groupes choisis parmi alkyle en C₁₋₆, NO₂ et halogène.

4. Procédé selon la revendication 1, dans lequel R^{b} et R^{c} sont, de façon indépendante, choisis parmi tert-butyldiméthylsilyle, hexyldiméthylsilyle et isopropyldiméthylsilyle.

5. Procédé selon la revendication 1, dans lequel R^{a} est tert-butyldiméthylsilyle.

6. Procédé selon la revendication 1, dans lequel l'acide est un acide mono-, di- ou tricarboxylique et le premier catalyseur est Pd/C, Pd(OH)₂/C ou (Ra)Ni.

7. Procédé selon la revendication 6, dans lequel l'acide est l'acide acétique ou l'acide formique et le premier catalyseur est Pd/C ou Pd(OH)₂/C.

8. Procédé selon la revendication 1, dans lequel la réaction est effectuée sous une pression de 68,95 à 689,48 kPa (10-100 psi) de H₂.

9. Procédé selon la revendication 7, dans lequel la réaction est effectuée sous une pression de 137,90 à 275,79 kPa (20-40 psi) de H₂.

10. Procédé pour la synthèse d'un composé de formule 6 : 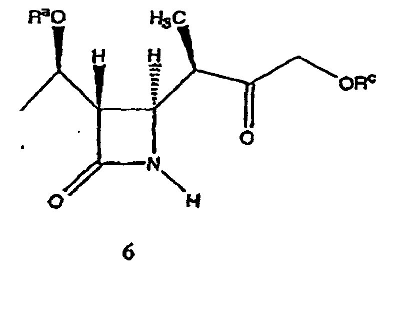 où:
R^{a} est
(1) un hydrogène,
(2) un alkyle en C₁₋₄ ou
(3) un groupe protecteur d'hydroxy ; et
R^{b} et R^{c} sont, de façon indépendante : un reste tri-organo-silyle choisi parmi tri(alkyl en C₁-C₆)silyle, phényldi(alkyl en C₁-C₆)silyle et diphénylmono(alkyl en C₁-C₆)silyle ; un alkyle inférieur, linéaire et ramifié, ayant 1 à 10 atomes de carbone ; un alcényle ou un alcynyle ayant 2 à 10 atomes de carbone ; un cycloalkyle ayant 3 à 6 atomes de carbone ; un cycloalkylalkyle dans lequel le fragment cycloalkyle contient 3 à 6 atomes de carbone et le fragment alkyle contient 1 à 10 atomes de carbone ; un alkylcycloalkyle dans lequel le fragment alkyle contient 1 à 10 atomes de carbone et le fragment cycloalkyle contient 3 à 6 atomes de carbone ; un aryle, tel que phényle et naphtyle; un aralkyle tel que benzyle, phénéthyle et analogue ; un hétérocyclyle (saturé et insaturé) comprenant des structures mono- et bicycliques ayant 5 à 10 atomes dans le squelette cyclique, un ou plusieurs des hétéroatomes étant choisis parmi l'oxygène, l'azote ou le soufre, tel que thiophène, imidazolyle, tétrazolyle, furyle et analogue ; un hétérocycloalkyle comprenant les fragments hétérocyclyle immédiatement ci-dessus et un fragment alkyle comprenant 1 à 10 atomes de carbone ; les espèces substituées dérivées des restes cités ci-dessus dans lesquelles les substituants sont choisis dans le groupe constitué par amino, hydroxy, cyano, carboxyle, nitro, chloro, bromo ou fluoro ; un alcoxy inférieur ayant 1 à 6 atomes de carbone; mercapto; un perhalogénoalkyle inférieur tel que trifluorométhyle ; un alkylthio inférieur ; guanidino ; amidino ; sulfamoyle ; les sulfamoyle, amidino et guanidino N-substitués dans lesquels le N-substituant est un alkyle inférieur ayant 1 à 6 atomes de carbone ou un aryle ayant 6-10 atomes de carbone ; COR ou CO₂R, où R est un alkyle inférieur ayant 1 à 6 atomes de carbone ou un aryle ayant 6-10 atomes de carbone ; où alkyle inférieur est un alkyle en C₁₋₆ et où R^{b} et R^{c} peuvent être identiques ou différents, mais où R^{b} doit obligatoirement pouvoir être éliminé en présence de R^{c} ;
comprenant : la combinaison du cétoester de formule 4 : 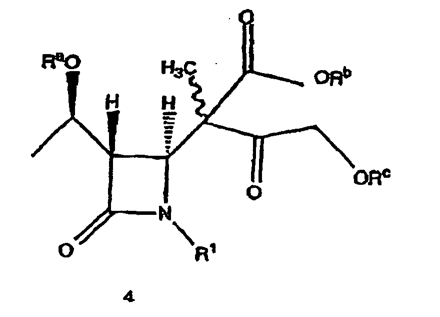 où R^{a}, R^{b} et R^{c} sont définis ci-dessus et R¹ est un groupe protecteur alkylsilyle correspondant à l'agent silylant employé ;
avec un acide et un premier catalyseur à une température de 0°C à 50°C sous une pression de 0 à 3447,38 kPa (0-500 psi) de H₂, pour obtenir un composé de formule 5 : 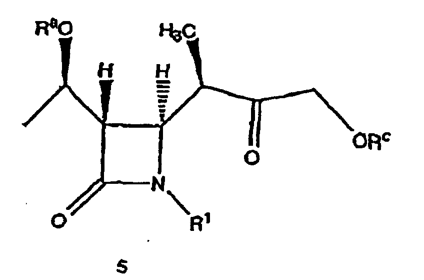 et la désilyladon sélective avec un agent désilylant à une température de -10°C à 50°C pour obtenir le composé de formule 6.

11. Procédé selon la revendication 10, dans lequel R^{a} est un groupe protecteur d'hydroxy et R^{b} et R^{c} sont, de façon indépendante, choisis dans le groupe constitué par benzyle, benzyle substitué, éthoxycarbonyle, t-butyloxycarbonyle, benzyloxycarbonyle, allyloxycarbonyle, fluorénylméthyloxycarbonyle, t-butyldiméthylsilyle et isopropyldiméthylsilyle, où ledit benzyle, quand il est substitué, est substitué par 1 à 3 groupes choisis parmi alkyle en C₁₋₆, NO₂ et halogène.

12. Procédé selon la revendication 11, dans lequel R^{a} est choisi dans le groupe constitué par tri(alkyl en C₁-C₆)silyle, phényldi(alkyl en C₁-C₆)silyle, diphénylmono(alkyl en C₁-C₆)silyle ; tert-butyldiméthylsilyle, benzyle substitué et non-substitué, allyle, triéthylsilyle, t-butyloxycarbonyle, o-nitro-benzyloxycarbonyle, p-nitrobenzyloxycarbonyle, 2,2,2-trichloro-éthyloxycarbonyle, benzyloxycarbonyle, allyloxycarbonyle et fluorénylméthyloxycarbonyle et R^{b} et R^{c} sont, de façon indépendante, benzyle, benzyle substitué, éthoxycarbonyle ou t-butyloxycarbonyle, où ledit benzyle, quand il est substitué, est substitué par 1 à 3 groupes choisis parmi alkyle en C₁₋₆, NO₂ et halogène.

13. Procédé selon la revendication 10, dans lequel R^{b} et R^{c} sont, de façon indépendante, choisis parmi tert-butyldiméthylsilyle, hexyldiméthylsilyle et isopropyldiméthylsilyle.

14. Procédé selon la revendication 10, dans lequel R^{a} est tert-butyldiméthylsilyle.

15. Procédé selon la revendication 10, dans lequel l'agent désilylant est choisi dans le groupe constitué par NaOH, KOH, l'acide est un acide mono-, di- ou tricarboxylique et un agent N-désilylant et le premier catalyseur est Pd/C, Pd(OH)₂/C ou (Ra)Ni.

16. Procédé selon la revendication 15, dans lequel l'agent désilylant est un agent N-désilylant choisi dans le groupe constitué par le fluorure de tétrabutylammonium (TBAF) ou la N-oxyde de 2-mercaptopyridine, l'acide est l'acide acétique ou l'acide formique et le premier catalyseur est Pd/C ou Pd(OH)₂/C.

17. Procédé selon la revendication 10, dans lequel la réaction est effectuée sous une pression de 68,95 à 689,48 kPa (10-100 psi) de H₂.

18. Procédé selon la revendication 15, dans lequel la réaction est effectuée sous une pression de 137,90 à 275,79 kPa (20-40 psi) de H₂.

19. Procédé pour la synthèse d'un composé de formule I : 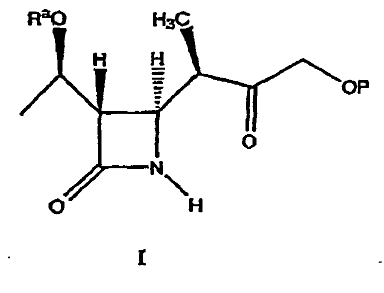 où:
R^{a} et P sont, de façon indépendante :
(1) un hydrogène,
(2) un alkyle en C₁₋₄ ou
(3) un groupe protecteur d'hydroxy ; et
comprenant:
(a) la réaction d'un cétoester de formule 1 : 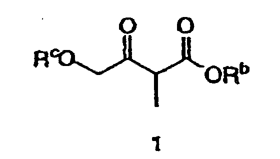 où R^{b} et R^{c} sont, de façon indépendante :
H, un reste tri-organo-silyle choisi parmi tri(alkyl en C₁-C₆)silyle, phényldi(alkyl en C₁-C₆)silyle et diphénylmono(alkyl en C₁-C₆)silyle ; un alkyle inférieur, linéaire et ramifié, ayant 1 à 10 atomes de carbone ; un alcényle ou un alcynyle ayant 2 à 10 atomes de carbone; un cycloallyle ayant 3 à 6 atomes de carbone ; un cycloalkylalkyle dans lequel le fragment cycloalkyie contient 3 à 6 atomes de carbone et le fragment alkyle contient 1 à 10 atomes de carbone ; un alkylcycloalkyle dans lequel le fragment alkyle contient 1 à 10 atomes de carbone et le fragment cycloalkyle contient 3 à 6 atomes de carbone ; un aryle, tel que phényle et naphtyle; un aralkyle tel que benzyle, phénéthyle et analogue ; un hétérocyclyle (saturé et insaturé) comprenant des structures mono- et bicycliques ayant 5 à 10 atomes dans le squelette cyclique, un ou plusieurs des hétéroatomes étant choisis parmi l'oxygène, l'azote ou le soufre, tel que thiophène, imidazolyle, tétrazolyle, furyle et analogue ; un hétérocycloalkyle comprenant les fragments hétérocyclyle immédiatement ci-dessus et un fragment alkyle comprenant 1 à 10 atomes de carbone ; les espèces substituées dérivées des restes cités ci-dessus dans lesquelles les substituants sont choisis dans le groupe constitué par amino, hydroxy, cyano, carboxyle, nitro, chloro, bromo ou fluoro ; un alcoxy inférieur ayant 1 à 6 atomes de carbone ; mercapto ; un perhalogénoalkyle inférieur tel que trifluorométhyle ; un alkylthio inférieur ; guanidino; amidino; sulfamoyle; les sulfamoyle, amidino et guanidino N-substitués dans lesquels le N-substituant est un alkyle inférieur ayant 1 à 6 atomes de carbone ou un aryle ayant 6-10 atomes de carbone; COR ou CO₂R, où R est un alkyle inférieur ayant 1 à 6 atomes de carbone ou un aryle ayant 6-10 atomes de carbone ; où alkyle inférieur est un alkyle en C₁₋₆ et où R^{b} et R^{c} peuvent être identiques ou différents, mais où R^{b} doit obligatoirement pouvoir être éliminé en présence de R^{c} ;
avec un composé de formule 2 : 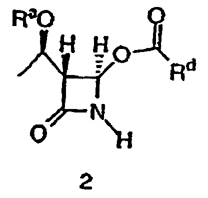
où R^{a} est défini ci-dessus et R^{d} est un alkyle en C₁₋₄ ;
en présence d'une première base à une température de 25°C à 60°C pour produire un produit d'addition de cétoester de formule 3 : 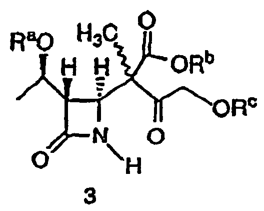
(b) la combinaison du composé de formule 3 avec un agent silylant et une seconde base pour produire le cétoester de formule 4 : 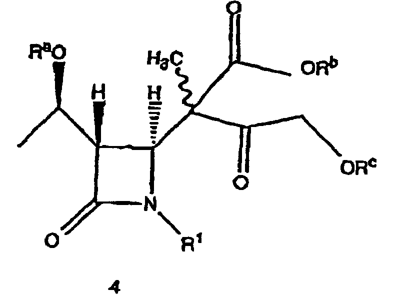 où R^{a}, R^{b} et R^{c} sont définis ci-dessus et R¹ est un groupe protecteur alkylsilyle correspondant à l'agent silylant employé ;
(c) la combinaison du cétoester de formule 4 isolé avec un acide et un premier catalyseur à une température de 0°C à 50°C sous une pression de 0 à 3447,38 kPa (0-500 psi) de H₂, pour obtenir un composé de formule 5 : 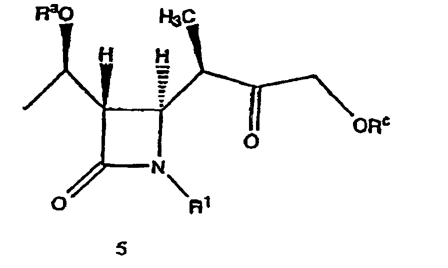
(d) la désilylation sélective avec un agent désilylant à une température de -10°C à 50°C pour obtenir le composé de formule 6. 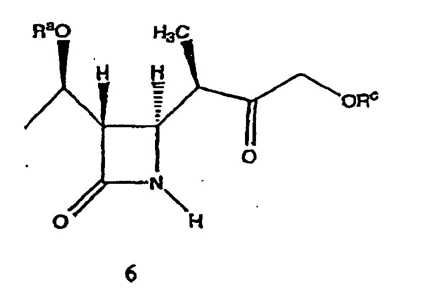
(e) la dissolution du composé de formule 6 dans un alcool et son hydrogénation sous une pression de 206,84 à 379,21 kPa (30-55 psi) de H₂, en présence d'un second catalyseur à une température de 0°C à 100°C, pour obtenir le composé I et
(f) la purification et l'isolement du composé I.

20. Procédé selon la revendication 19, dans lequel R^{a} est un groupe protecteur d'hydroxy et R^{b} et R^{c} sont, de façon indépendante, choisis dans le groupe constitué par benzyle, benzyle substitué, éthoxycarbonyle, t-butyloxycarbonyle, benzyloxycarbonyle, allyloxycarbonyle, fluorénylméthyloxycarbonyle, t-butyldiméthylsilyle et isopropyldiméthylsilyle, où ledit benzyle, quand il est substitué, est substitué par 1 à 3 groupes choisis parmi alkyle en C₁₋₆, NO₂ et halogène.

21. Procédé selon la revendication 20, dans lequel R^{a} est choisi dans le groupe constitué par tri(alkyl cn C₁-C₆)silyle, phényldi(alkyl en C₁-C₆)silyle, diphénylmono(alkyl en C₁-C₆)silyle ; tert-butyldiméthylsilyle, benzyle substitué et non-substitué, allyle, triéthylsilyle, t-bufyloxycarbonyle, o-nitro-benzyloxycarbonyle, p-nitrobenzyloxycarbonyle, 2,2,2-trichloro-éthyloxycarbonyle, benzyloxycarbonyle, allyloxycarbonyle et fluorénylméthyloxycarbonyle et R^{b} et R^{c} sont, de façon indépendante, H, benzyle, benzyle substitué, éthoxycarbonyle ou t-butyloxycarbonyle, où ledit benzyle, quand il est substitué, est substitué par 1 à 3 grouper choisis parmi alkyle en C₁₋₆, NO₂ et halogène.

22. Procédé selon la revendication 19, dans lequel R^{b} et R^{c} sont, de façon indépendante, choisis parmi tert-butyldiméthylsilyle, bexyldiméthylsilyle et isopropyldiméthylsilyle.

23. Procédé selon la revendication 19, dans lequel R^{a} est tert-butyldiméthyisilyle.

24. Procédé selon la revendication 19, dans lequel la réaction à l'étape (c) est effectuée sous une pression de 68,95 à 689,48 kPa (10-100 psi) de H₂.

25. Procédé selon la revendication 24, dans lequel la réaction à l'étape (c) est effectuée sous une pression de 137,90 à 275,79 kPa (20-40 psi) de H₂.

26. Procédé selon la revendication 19, dans lequel les première et seconde bases sont choisies dans le groupe constitué par le carbonate de potassium, le carbonate de calcium, le bicarbonate, la diisopropyléthylamine, la triéthylamine, la diméthyléthylamine, la diméthylpentylamine et le diazabicycloundécane ; l'acide silylant est choisi dans le groupe constitué par les chlorures de trialkylsilyle, les iodures de trialkylsilyle et les triflates et l'agent désilylant est choisi dans le groupe constitué par NaOH, KOH et un agent N-désilylant.

27. Procédé selon la revendication 26, dans lequel la première base est le carbonate de potassium ou le diazabicycloundécanc, la deuxième base est la triéthylamine ou le diazabicycloundécane ; l'acide silylant est le triflate de triméthylsilyle (TMSOTf), le triflate de tert-butyldiméthylsilyle (TBSOTf), le triflate de triéthylsilyle (TESOTf) ou chlorure de tert-butyldiméthylsilyle / iodure de sodium (TBSCl/NaI) ; et l'agent désilylant est un agent N-désilylant choisi dans le groupe constitué par le fluorure de tétrabutylammonium (IBAF) ou le N-oxydc de 2-mercaptopyridine.

28. Procédé selon la revendication 19, dans lequel l'acide est un acide mono-, di- ou tricarboxylique ; l'alcool est le méthanol, l'éthanol, le butanol, le pentanol, le 1-propanol ou le 2-propanol et le premier et le second catalyseurs sont Pd/C, Pd(OH)₂/C ou (Ra)Ni.

29. Procédé selon la revendication 28, dans lequel l'acide est l'acide acétique ou l'acide formique ; l'alcool est le méthanol ou l'éthanol et le premier et le second catalyseurs sont Pd/C ou Pd(OH)₂/C.
